# EUROPEAN PATENT SPECIFICATION

(11) **EP 4 336 512 B1**
(45) Date of publication and mention of the grant of the patent: **02.12.2026**
(21) Application number: 22881430.7
(22) Date of filing: 17.10.2022
(51) Int. Cl.: G16H 20/30, G16H 10/60, A63B 71/06, A63B 24/00, G16Y 40/20, G16Y 10/60, G16H 50/20, G16H 40/63, G16H 40/67

(54) **METHOD AND DEVICE FOR PROVIDING AN EXERCISE PROGRAM TO A USER**
VERFAHREN UND GERÄT ZUR BEREITSTELLUNG EINES ÜBUNGSPROGRAMMS FÜR EINEN BENUTZER
PROCÉDÉ ET DISPOSITIF POUR FOURNIR UN PROGRAMME D'EXERCICE À UN UTILISATEUR

(30) Priority: 15.10.2021 KR 20210137285; 08.07.2022 KR 20220084402
(43) Date of publication of application: 13.03.2024
(73) Proprietor: Samsung Electronics Co., Ltd., Suwon-si, Gyeonggi-do 16677 (KR)
(72) Inventor: KIM, Sungcheol, Suwon-si Gyeonggi-do 16677 (KR); KIM, Kyungrock, Suwon-si Gyeonggi-do 16677 (KR); SEO, Keehong, Suwon-si Gyeonggi-do 16677 (KR); LIM, Bokman, Suwon-si Gyeonggi-do 16677 (KR)
(74) Representative: Gulde & Partner
(86) International application number: PCT/KR2022/015692
(87) International publication number: WO 2023/063803

(56) References cited:
- WO-A1-2020/208945
- JP-B2- 5 237 227
- KR-A- 20160 054 325
- KR-A- 20160 091 694
- KR-A- 20210 030 855
- US-A1- 2021 121 741

## Description

### Technical Field

The present application relates to a technology for providing an exercise program to a user.

### Background Art

With the onset of aging societies, a growing number of people experience inconvenience and pain in walking from weakened muscular strength or joint problems due to aging, and there is growing interest in walking assist devices that enable the elderly with weakened muscular strength or patients with muscular joint discomfort to walk with ease. In that regard US 2021/121741 A1 discloses a balance training method using a wearable device providing a walking assist function. US 2019/160321 Al discloses a biomechanical assistive device for generating and providing torque assistance to a user.

### DISCLOSURE OF THE INVENTION

### Technical Goals

According to a first aspect of the present invention, there is provided an electronic device including: a communication module (comprising communication circuitry) configured to exchange data with an external device; and at least one processor configured to control the electronic device, wherein the at least one processor is configured to:
receive target exercise information from a user of the electronic device, the target exercise information including at least one of target exercise time information and target exercise section information including an exercise route; based on at least one of the target exercise time information and the target exercise section information and current values of gait assessment items, determine an optimal value of a control parameter that satisfies preset target values of the gait assessment items, wherein the optimal value is based on topographic information on the exercise route and wherein the control parameter being a parameter for adjusting at least one of a magnitude, torque direction and timing, an offset angle between joint angles, or sensitivity of a state factor to the joint angles or combination thereof; acquire one or more recommended exercise program based on the optimal value of the control parameter; determine a target exercise program among the one or more recommended exercise program; transmit information on the target exercise program to a wearable device worn by the user; receive sensing information from the wearable device while the target exercise program is being executed by the wearable device; and provide feedback information upon execution of the target exercise program to the user based on the sensing information.

The items for assessing a gait may include one or more of a step length, gait speed, gait symmetry, and gait rhythm.

The processor may acquire the current values of the gait assessment items based on previous gait information sensed while the user was performing a previous exercise program.

The processor may transmit information on a test exercise program for acquiring the current values of the gait assessment items to the wearable device, receive test sensing information upon execution of the test exercise program from the wearable device, and acquire the current values of the gait assessment items based on the test sensing information.

The processor may determine the optimal value of the control parameter such that the target values of the gait assessment items may be satisfied within a range that satisfies a set objective function.

The processor may transmit the optimal value of the control parameter to the server, and receive, from the server, one or more recommended exercise program determined by the server based on the optimal value of the control parameter.

The processor may determine one or more recommended exercise program corresponding to the optimal value among a plurality of stored exercise programs.

The processor may determine the one or more recommended exercise program corresponding to the optimal value among the plurality of stored exercise programs based on a history of exercise programs performed by the user.

The at least one recommended exercise program may be different from a previous exercise program performed by the user on the exercise section.

The processor may determine whether the target values of the assessment items are being satisfied based on the sensing information, when the target values are not satisfied, determine a requested action to satisfy the target values, and provide the feedback information including the requested action to the user.

The processor may transmit the feedback information to an additional electronic device, and the feedback information may be output by the additional electronic device.

The additional electronic device may be any one of an earphone, a glasses-type electronic device, or a watch-type electronic device, for example and without limitation.

The processor may receive a heart rate of the user from the additional electronic device as the sensing information.

The target exercise section information may include information on a start point, an end point, and detailed routes between the start point and the end point.

An expected gait age may be provided to the user in response to the target exercise program being performed normally.

The target values of the gait assessment items may be set based on a target exercise goal selected by the user among a plurality of exercise goals.

The plurality of exercise objectives may include two or more of improving gait ability, improving gait posture, improving cardiovascular health, and improving muscular strength.

According to a second aspect of the present invention, there is provided a method performed by an electronic device, wherein the method includes : receiving target exercise information from a user of the electronic device, the target exercise information including at least one of target exercise time information and target exercise section information including an exercise route; based on the at least one of the target exercise time information and the target exercise section information and current values of gait assessment items, determining an optimal value of a control parameter that satisfies preset target values of the gait assessment items, wherein the optimal value is based on topographic information on the exercise route and wherein the control parameter being a parameter for adjusting at least one of a magnitude, torque direction and timing, an offset angle between joint angles, or sensitivity of a state factor to the joint angles or combination thereof; acquiring one or more recommended exercise program based on the optimal value of the control parameter; determining a target exercise program among the one or more recommended exercise program; transmitting information on the target exercise program to a wearable device worn by the user; receiving sensing information from the wearable device while the target exercise program is being executed by the wearable device; and providing feedback information upon execution of the target exercise program to the user based on the sensing information.

The at least one recommended exercise program may be different from a previous exercise program performed by the user on the exercise section

.

### Effects

According to certain embodiments, various exercise programs may be provided to a user through a wearable device.

According to certain embodiments, a user wearing the wearable device may feel as if a corresponding exercise section is a different exercise section through performing a different exercise program a number of times, even when the user repeatedly performs an exercise in the same exercise section.

### BRIEF DESCRIPTION OF DRAWINGS

FIG. 1 is a diagram illustrating a system for providing an exercise program to a user according to an example embodiment;
FIG. 2 is a block diagram illustrating an electronic device in a network environment according to an example embodiment;
FIGS. 3a to 3d are diagrams illustrating a wearable device according to embodiments;
FIG. 4 is a diagram illustrating a wearable device communicating with an electronic device according to an example embodiment;
FIGS. 5 and 6 are diagrams illustrating a method of outputting a torque by a wearable device according to certain example embodiments;
FIG. 7 is a block diagram illustrating optimization of parameter values of a wearable device according to an example embodiment;
FIG. 8 is a flowchart illustrating a method of providing an exercise program to a user according to an example embodiment;
FIG. 9 is a flowchart illustrating a method of acquiring current values of gait assessment items according to an example embodiment;
FIG. 10 is a flowchart illustrating a method of determining an optimal value of a control parameter according to an example embodiment;
FIG. 11 is a diagram illustrating a method of determining recommended exercise programs based on target exercise information according to an example embodiment;
FIG. 12 is a flowchart illustrating a method of acquiring recommended exercise programs through a server according to an example embodiment;
FIG. 13 is a flowchart illustrating a method of providing feedback information to a user according to an example embodiment; and
FIG. 14 is a diagram illustrating a configuration of a server according to an example embodiment.

### BEST MODE FOR CARRYING OUT THE INVENTION

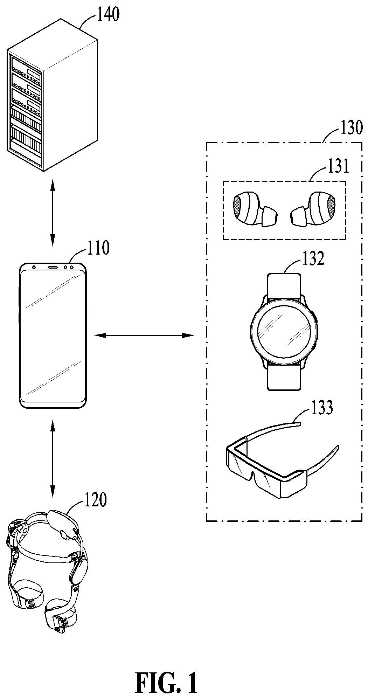

FIG. 1 is a diagram illustrating a system for providing an exercise program to a user according to an example embodiment.

According to an example embodiment, a system for providing an exercise program to a user may include an electronic device 110, a wearable device 120, an additional device 130, and a server 140.

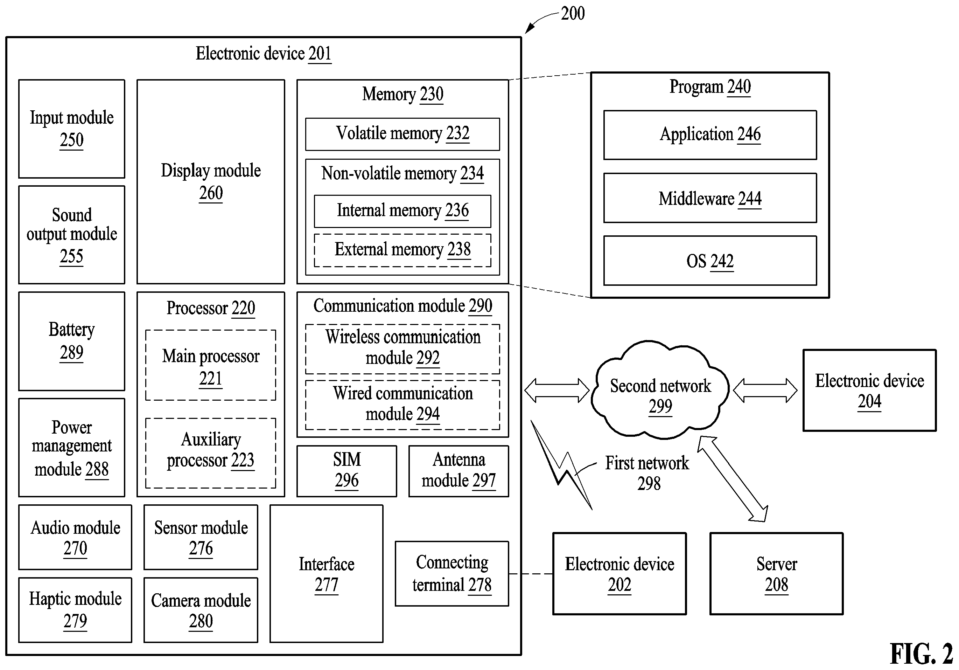

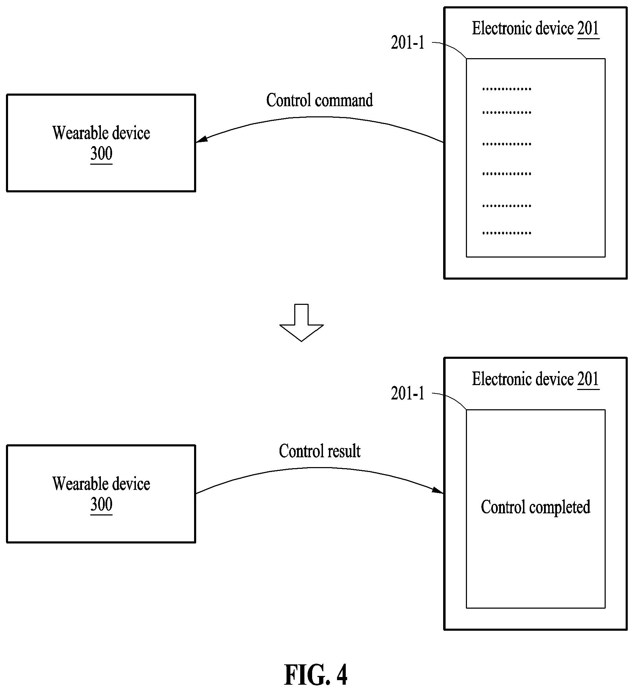

According to an example embodiment, the electronic device 110 may be a user terminal connectable to the wearable device 120 using short-range wireless communication. For example, the electronic device 110 may transmit a control signal for controlling the wearable device 120 to the wearable device 120. The electronic device 110 will be described in detail below with reference to FIG. 2, and transmission of the control signal will be described in detail below with reference to FIG. 4.

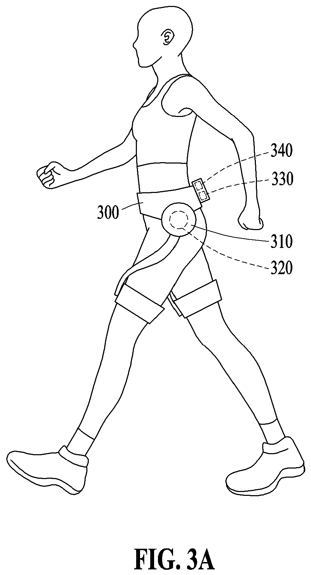

According to an example embodiment, the wearable device 120 may provide an assistance force to assist walking or a resistance force to hinder walking, to a user wearing the wearable device 120. The resistance force may be provided to the user to assist the user in doing an exercise. By controlling values of various control parameters used in the wearable device 120, the assistance force or the resistance force output by the wearable device 120 may be controlled. A structure and a driving method of the wearable device 120 will be described in detail below with reference to FIGS. 3a to 7.

According to an example embodiment, the electronic device 110 may be connected, directly or indirectly, to the additional device 130 (e.g., a wireless earphone 131, a smartwatch 132, or smart glasses 133) using short-range wireless communication. For example, the electronic device 110 may output information indicating a state of the electronic device 110 or a state of the wearable device 120 to the user through the additional device 130. For example, feedback information on a walking state of the user wearing the wearable device 120 may be output through a haptic device, a speaker device, and a display device of the additional device 130.

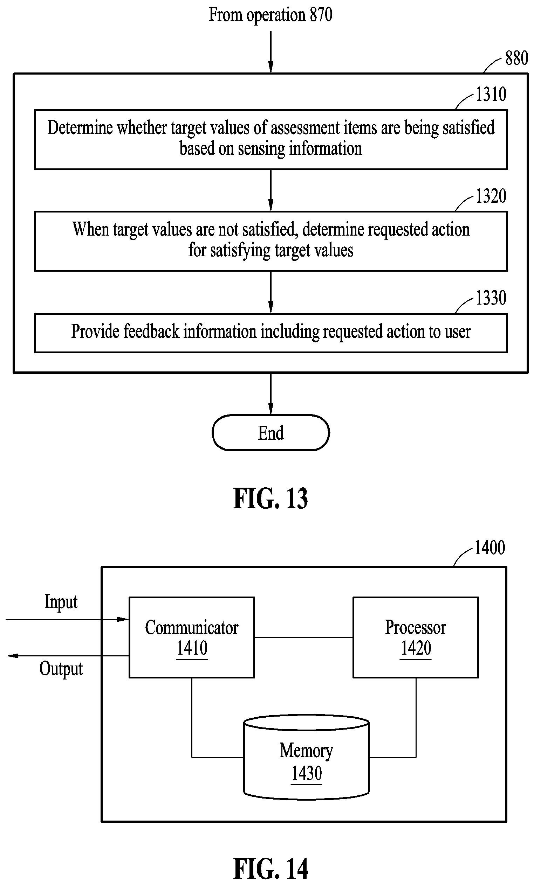

According to an example embodiment, the electronic device 110 may be connected, directly or indirectly, to the server 140 using short-range wireless communication or cellular communication. For example, the server 140 may include a database in which information on a plurality of exercise programs that can be provided to a user through the wearable device 120 is stored. For example, the server 140 may manage a user account for a user of the electronic device 110 or the wearable device 120. The server 140 may store and manage an exercise program performed by the user and a result of the exercise program in association with the user account. An example configuration of the server 140 will be described in detail below with reference to FIG. 14.

According to an example embodiment, the system may provide the user with an exercise program to achieve an exercise objective in various exercise environments desired by the user. For example, the exercise objective of the user may be preset, and may include, for example, improvement of gait ability, improvement of gait posture, improvement of cardiovascular health, and improvement of muscular strength. Based on the exercise objective, the user may designate a predetermined exercise environment each time an exercise is performed. For example, the user may designate a target exercise section and a target exercise time as an exercise environment through the electronic device 110 before performing an exercise. The electronic device 110 may determine values of control parameters of the wearable device 120 that may satisfy the exercise environment based on the set exercise objective. For example, the control parameters may include parameters for adjusting at least one of a magnitude, a direction, or a torque timing or combination thereof to be output through the wearable device 120, an offset angle between joint angles of the wearable device 120, and sensitivity of a state factor to the joint angles. The electronic device 110 may acquire one or more exercise program based on the determined values of the control parameters.

According to an example embodiment, the exercise program may relate to a method of providing an assistance force or a resistance force provided to a user wearing the wearable device 120 in a set exercise environment. For example, the exercise program may provide the user with the same assistance force or the same resistance force within an entire exercise time. In another example, the exercise program may divide the entire exercise time into a plurality of sections, and provide different assistance forces or resistance forces to the user in the plurality of sections. For example, an output timing of the assistance force or the resistance force output through the exercise program may vary according to the target exercise time and the exercise objective.

According to an example embodiment, the plurality of exercise programs may be converted into a database and stored in the electronic device 110 or in the server 140. For example, the electronic device 110 or the server 140 may recommend one or more of the plurality of exercise programs to the user based on the determined values of the control parameters. For example, the electronic device 110 or the server 140 may determine an exercise program to be recommended to the user based on a history of exercise performed by the user. Accordingly, a new exercise program may be recommended to the user even when the user is exercising in the same exercise environment, and the user may feel as if they are performing a different exercise than the existing exercise by performing the new exercise program.

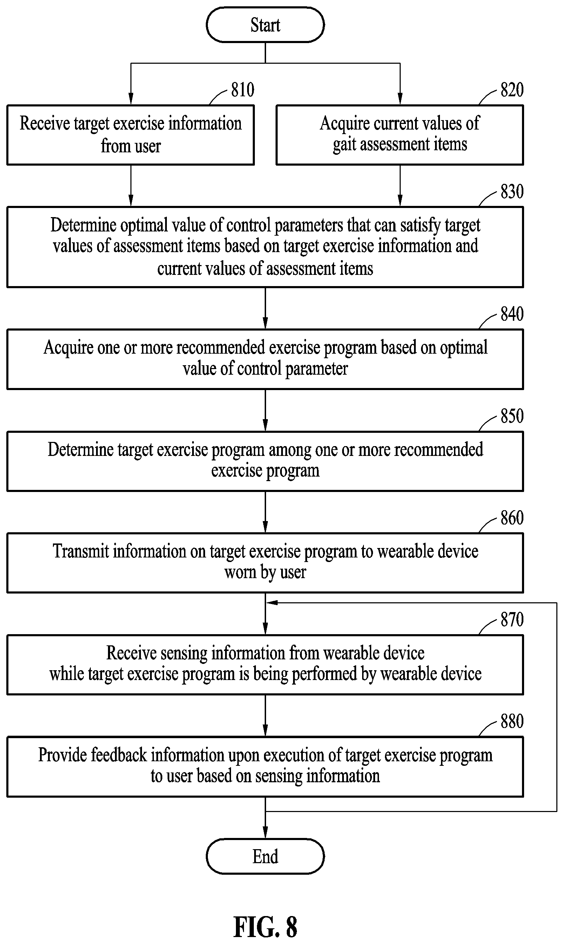

A method of providing an exercise program to a user will be described in detail below with reference to FIGS. 8 to 13.

FIG. 2 is a block diagram illustrating an electronic device in a network environment according to embodiments.

FIG. 2 is a block diagram illustrating an electronic device 201 (e.g., the electronic device 110 of FIG. 1) in a network environment 200 according to embodiments. Referring to FIG. 2, the electronic device 201 in the network environment 200 may communicate with an electronic device 202 via a first network 298 (e.g., a short-range wireless communication network), or communicate with at least one of an electronic device 204 and a server 208 via a second network 299 (e.g., a long-range wireless communication network). According to an example embodiment, the electronic device 201 may communicate with the electronic device 204 via the server 208. According to an example embodiment, the electronic device 201 may include a processor 220, a memory 230, an input module 250, a sound output module 255, a display module 260, an audio module 270, and a sensor module 276, an interface 277, a connecting terminal 278, a haptic module 279, a camera module 280, a power management module 288, a battery 289, a communication module 290, a subscriber identification module (SIM) 296, or an antenna module 297. In some embodiments, at least one (e.g., the connecting terminal 278) of the above components may be omitted from the electronic device 201, or one or more other components may be added in the electronic device 201. In some embodiments, some (e.g., the sensor module 276, the camera module 280, or the antenna module 297) of the components may be integrated as a single component (e.g., the display module 260). Each antenna module herein comprises at least one antenna, and each sensor module herein comprises circuitry and/or at least one sensor.

The processor 220 may execute, for example, software (e.g., a program 240) to control at least one other component (e.g., a hardware or software component) of the electronic device 201 connected, directly or indirectly, to the processor 220, and may perform various data processing or computation. According to an example embodiment, as at least a part of data processing or computation, the processor 220 may store a command or data received from another component (e.g., the sensor module 276 or the communication module 290) in a volatile memory 232, process the command or the data stored in the volatile memory 232, and store resulting data in a non-volatile memory 234. According to an example embodiment, the processor 220 may include a main processor 221 (e.g., a central processing unit (CPU) or an application processor (AP)) or an auxiliary processor 223 (e.g., a graphics processing unit (GPU), a neural processing unit (NPU), an image signal processor (ISP), a sensor hub processor, or a communication processor (CP)) that is operable independently of, or in conjoint with the main processor 221. For example, when the electronic device 201 includes the main processor 221 and the auxiliary processor 223, the auxiliary processor 223 may be adapted to consume less power than the main processor 221 or to be specific to a specified function. The auxiliary processor 223 may be implemented separately from the main processor 221 or as a part of the main processor 221.

The auxiliary processor 223 may control at least some of functions or states related to at least one (e.g., the display module 260, the sensor module 276, or the communication module 290) of the components of the electronic device 201, instead of the main processor 221 while the main processor 221 is in an inactive (e.g., sleep) state or along with the main processor 221 while the main processor 221 is in an active state (e.g., executing an application). According to an example embodiment, the auxiliary processor 223 (e.g., an ISP or a CP) may be implemented as a portion of another component (e.g., the camera module 280 or the communication module 290) that is functionally related to the auxiliary processor 223. According to an example embodiment, the auxiliary processor 223 (e.g., an NPU) may include a hardware structure specified for artificial intelligence (AI) model processing. An AI model may be generated by machine learning. The machine learning may be performed by, for example, the electronic device 201, in which artificial intelligence is performed, or performed via a separate server (e.g., the server 208). Learning algorithms may include, but are not limited to, for example, supervised learning, unsupervised learning, semi-supervised learning, or reinforcement learning algorithms. The AI model may include a plurality of artificial neural network layers. An artificial neural network may include, for example, a deep neural network (DNN), a convolutional neural network (CNN), a recurrent neural network (RNN), a restricted Boltzmann machine (RBM), a deep belief network (DBN), and a bidirectional recurrent deep neural network (BRDNN), a deep Q-network, or a combination of two or more thereof, but is not limited thereto. The AI model may additionally or alternatively include a software structure other than the hardware structure.

The memory 230 may store various pieces of data used by at least one component (e.g., the processor 220 or the sensor module 276) of the electronic device 201. The various pieces of data may include, for example, software (e.g., the program 240) and input data or output data for a command related thereto. The memory 230 may include the volatile memory 232 or the non-volatile memory 234.

The program 240 may be stored as software in the memory 230, and may include, for example, an operating system (OS) 242, middleware 244, or an application 246.

The input module 250 may receive, from outside (e.g., a user) the electronic device 201, a command or data to be used by another component (e.g., the processor 220) of the electronic device 201. The input module 250 may include, for example, a microphone, a mouse, a keyboard, a key (e.g., a button), or a digital pen (e.g., a stylus pen), and typically comprises input circuitry.

The sound output module 255 may output a sound signal to the outside of the electronic device 201. The sound output module 255 may include, for example, a speaker or a receiver. The speaker may be used for general purposes, such as playing multimedia or playing a recording. The receiver may be used to receive an incoming call. According to an example embodiment, the receiver may be implemented separately from the speaker or as a part of the speaker.

The display module 260, comprising a display, may visually provide information to the outside (e.g., a user) of the electronic device 201. The display module 260 may include, for example, a control circuit for controlling a display, a hologram device, or a projector and control circuitry to control its corresponding one of the display, the hologram device, and the projector. According to an example embodiment, the display device 260 may include a touch sensor adapted to sense a touch, or a pressure sensor adapted to measure an intensity of a force incurred by the touch.

The audio module 270 may convert sound into an electrical signal or vice versa. According to an example embodiment, the audio module 270 may acquire the sound via the input module 250 or output the sound via the sound output module 255 or an external electronic device (e.g., an electronic device 202 such as a speaker or a headphone) directly or wirelessly connected to the electronic device 201.

The sensor module 276 may detect an operational state (e.g., power or temperature) of the electronic device 201 or an environmental state (e.g., a state of a user) external to the electronic device 201, and generate an electrical signal or data value corresponding to the detected state. According to an example embodiment, the sensor module 276 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 277 may support one or more specified protocols to be used by the electronic device 201 to couple with the external electronic device (e.g., the electronic device 202) directly (e.g., by wire) or wirelessly. According to an example embodiment, the interface 277 may include, for example, a high-definition multimedia interface (HDMI), a universal serial bus (USB) interface, a secure digital (SD) card interface, or an audio interface.

The connecting terminal 278 may include a connector via which the electronic device 201 may physically connect to an external electronic device (e.g., the electronic device 202). According to an example embodiment, the connecting terminal 278 may include, for example, an HDMI connector, a USB connector, an SD card connector, or an audio connector (e.g., a headphone connector).

The haptic module 279 may convert an electric signal into a mechanical stimulus (e.g., a vibration or a movement) or an electrical stimulus which may be recognized by a user via their tactile sensation or kinesthetic sensation. According to an example embodiment, the haptic module 279 may include, for example, a motor, a piezoelectric element, or an electric stimulator.

The camera module 280 may capture a still image and moving images. According to an example embodiment, the camera module 280 may include one or more lenses, image sensors, ISPs, or flashes.

The power management module 288 may manage power supplied to the electronic device 201. According to an example embodiment, the power management module 288 may be implemented as, for example, at least a part of a power management integrated circuit (PMIC).

The battery 289 may supply power to at least one component of the electronic device 201. According to an example embodiment, the battery 289 may include, for example, a primary cell which is not rechargeable, a secondary cell which is rechargeable, or a fuel cell.

The communication module 290 may support establishing a direct (e.g., wired) communication channel or a wireless communication channel between the electronic device 201 and the external electronic device (e.g., the electronic device 202, the electronic device 204, or the server 208) and performing communication via the established communication channel. The communication module 290 may include one or more CPs that are operable independently from the processor 220 (e.g., an AP) and that support direct (e.g., wired) communication or wireless communication. According to an example embodiment, the communication module 290 may include a wireless communication module 292 (e.g., a cellular communication module, a short-range wireless communication module, or a global navigation satellite system (GNSS) communication module) or a wired communication module 294 (e.g., a local region network (LAN) communication module, or a power line communication (PLC) module). A corresponding one of these communication modules may communicate with the external electronic device, for example, the electronic device 204, via the first network 298 (e.g., a short-range communication network, such as Bluetooth^{™}, wireless-fidelity (Wi-Fi) direct, or infrared data association (IrDA)) or the second network 299 (e.g., a long-range communication network, such as a legacy cellular network, a 5G network, a next-generation communication network, the Internet, or a computer network (e.g., a LAN or a wide area network (WAN))). These various types of communication modules may be implemented as a single component (e.g., a single chip), or may be implemented as multiple components (e.g., multiple chips) separate from each other. The wireless communication module 292 may identify and authenticate the electronic device 201 in a communication network, such as the first network 298 or the second network 299, using subscriber information (e.g., international mobile subscriber identity (IMSI)) stored in the SIM 296.

The wireless communication module 292 may support a 5G network after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 292 may support a high-frequency band (e.g., a mmWave band) to achieve, e.g., a high data transmission rate. The wireless communication module 292 may support various technologies for securing performance on a high-frequency band, such as, e.g., beamforming, massive multiple-input and multiple-output (MIMO), full dimensional MIMO (FD-MIMO), an array antenna, analog beam-forming, or a large scale antenna. The wireless communication module 292 may support various requirements specified in the electronic device 201, an external electronic device (e.g., the electronic device 204), or a network system (e.g., the second network 299). According to an example embodiment, the wireless communication module 292 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 297 may transmit or receive a signal or power to or from the outside (e.g., the external electronic device) of the electronic device 201. According to an example embodiment, the antenna module 297 may include an antenna including a radiating element including a conductive material or a conductive pattern formed in or on a substrate (e.g., a printed circuit board (PCB)). According to an example embodiment, the antenna module 297 may include a plurality of antennas (e.g., array antennas). In such a case, at least one antenna appropriate for a communication scheme used in a communication network, such as the first network 298 or the second network 299, may be selected by, for example, the communication module 290 from the plurality of antennas. The signal or power may be transmitted or received between the communication module 290 and the external electronic device via the at least one selected antenna. According to an example embodiment, another component (e.g., a radio frequency integrated circuit (RFIC)) other than the radiating element may be additionally formed as a part of the antenna module 297.

According to an example embodiment, the antenna module 297 may form a mmWave antenna module. According to an example embodiment, the mmWave antenna module may include a PCB, an RFIC disposed on a first surface (e.g., a bottom surface) of the PCB or adjacent to the first surface and capable of supporting a designated a high-frequency band (e.g., the mmWave band), and a plurality of antennas (e.g., array antennas) disposed on a second surface (e.g., a top or a side surface) of the PCB, or adjacent to the second surface and capable of transmitting or receiving signals in the designated high-frequency band.

At least some of the above-described components may be coupled mutually and exchange signals (e.g., commands or data) therebetween via an inter-peripheral communication scheme (e.g., a bus, general purpose input and output (GPIO), serial peripheral interface (SPI), or mobile industry processor interface (MIPI)).

According to an example embodiment, commands or data may be transmitted or received between the electronic device 201 and the external electronic device 204 via the server 208 coupled with the second network 299. Each of the external electronic devices (e.g., the electronic device 202 and/or 204) may be a device of the same type as or a different type from the electronic device 201. According to an example embodiment, all or some of operations to be executed by the electronic device 201 may be executed at one or more external electronic devices (e.g., the external devices 202 and/or 204, and the server 208). For example, if the electronic device 201 needs to perform a function or a service automatically, or in response to a request from a user or another device, the electronic device 201, instead of, or in addition to, executing the function or the service, may request one or more external electronic devices to perform at least portion of the function or service. The one or more external electronic devices receiving the request may perform the at least part of the function or service, or an additional function or an additional service related to the request, and may transfer a result of the performance to the electronic device 201. The electronic device 201 may provide the result, with or without further processing the result, as at least part of a response to the request. To that end, cloud computing, distributed computing, mobile edge computing (MEC), or client-server computing technology may be used, for example. The electronic device 201 may provide ultra low-latency services using, e.g., distributed computing or MEC. In an example embodiment, the external electronic device (e.g., the electronic device 202 and/or 204) may include an Internet-of-things (IoT) device. The server 208 may be an intelligent server using machine learning and/or a neural network. According to an example embodiment, the external electronic device 204 or the server 208 may be included in the second network 299. The electronic device 201 may be applied to intelligent services (e.g., a smart home, a smart city, a smart car, or healthcare) based on 5G communication technology or IoT-related technology.

The electronic device according to embodiments may be one of various types of electronic devices. The electronic device may include, for example, a portable communication device (e.g., a smartphone), a computer device, a portable multimedia device, a portable medical device, a camera, a wearable device, or a home appliance device. According to an embodiment, the electronic device is not limited to those described above.

It should be appreciated that various embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things unless the relevant context clearly indicates otherwise. As used herein, "A or B", "at least one of A and B", "at least one of A or B", "A, B or C", "at least one of A, B and C", and "A, B, or C," each of which may include any one of the items listed together in the corresponding one of the phrases, or all possible combinations thereof. Terms such as "first", "second", or "first" or "second" may simply be used to distinguish the component from other components in question, and do not limit the components in other aspects (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., by wire), wirelessly, or via at least a third element.

As used in connection with various embodiments, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an example embodiment, the module may be implemented in a form of an application-specific integrated circuit (ASIC).

Various embodiments of the present disclosure as set forth herein may be implemented as software (e.g., the program 240) including one or more instructions that are stored in a storage medium (e.g., an internal memory 236 or an external memory 238) that is readable by a machine (e.g., the electronic device 201). For example, a processor (e.g., the processor 220) of the machine (e.g., the electronic device 201) may invoke at least one of the one or more instructions stored in the storage medium, and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an example embodiment, a method according to various embodiments may be included and provided in a computer program product. The computer program product may be traded as a product between a seller and a buyer. The computer program product may be distributed in the form of a machine-readable storage medium (e.g., a compact disc read-only memory (CD-ROM)), or be distributed (e.g., downloaded or uploaded) online via an application store (e.g., PlayStore^{™}), or between two user devices (e.g., smartphones) directly. If distributed online, at least part of the computer program product may be temporarily generated or at least temporarily stored in the machine-readable storage medium, such as memory of the manufacturer's server, a server of the application store, or a relay server.

According to embodiments, each component (e.g., a module or a program) of the above-described components may include a single entity or multiple entities, and some of the multiple entities may be separately disposed in different components. According to embodiments, one or more of the above-described components may be omitted, or one or more other components may be added. Alternatively or additionally, a plurality of components (e.g., modules or programs) may be integrated into a single component. In such a case, according to embodiments, the integrated component may still perform one or more functions of each of the plurality of components in the same or similar manner as they are performed by a corresponding one of the plurality of components before the integration. According to embodiments, operations performed by the module, the program, or another component may be carried out sequentially, in parallel, repeatedly, or heuristically, or one or more of the operations may be executed in a different order or omitted, or one or more other operations may be added.

FIGS. 3a to 3d are diagrams illustrating a wearable device according to embodiments.

Referring to FIGS. 3a to 3d, a wearable device 300 (e.g., the wearable device 120 of FIG. 1) may be worn by a user to assist the user in a gait. For example, the wearable device 300 may be a device that assists a user in walking. Also, the wearable device 300 may be an exercise device that assists the user in walking and provides an exercise function by providing a resistance force to the user. For example, the resistance force provided to the user, such as a force output by a device such as a motor, may be actively applied to the user. In another example, the resistance force, such as a frictional force, may not be actively applied to the user, but may hinder a movement of the user. The resistance force may be referred to as an exercise load.

Although FIGS. 3a to 3d show the wearable device 300 as a hip-type, the type of the wearable device is not limited thereto, and the wearable device may be a type supporting all of the lower extremities or a type supporting a part of the lower extremities. In addition, the wearable device may be in any one of a form supporting a part of the lower extremities, a form supporting up to the knee, a form supporting up to the ankle, and a form supporting the whole body.

The embodiments described with reference to FIGS. 3a to 3d may be applied to a hip-type, but are not limited thereto, and may be applied to various types of wearable devices.

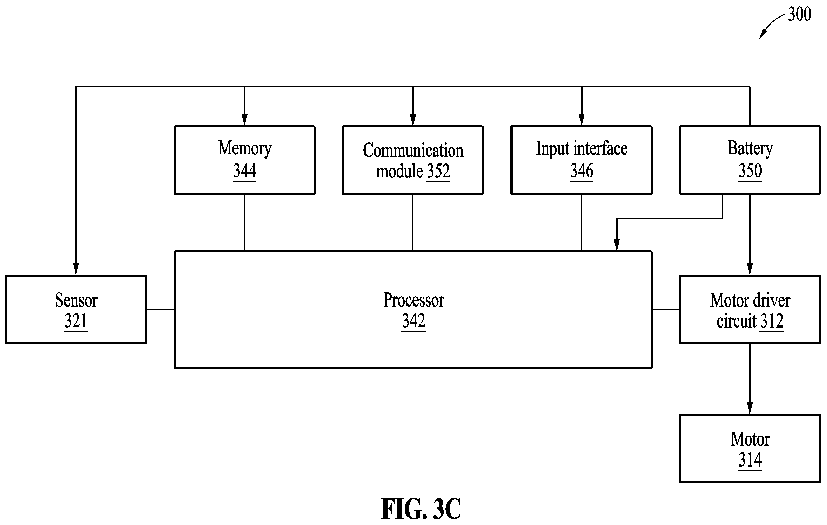

According to an example embodiment, the wearable device 300 may include a driver 310, a sensor 320 (e.g., 321 and/or 321-1 in Figs. 3c-3d), an inertial measurement unit (IMU) 330, a controller 340, a battery 350, and a communication module 352 comprising communication circuitry. For example, the IMU 330 and the controller 340 may be arranged in a main frame of the wearable device 300. In another example, the IMU 330 and the controller 340 may be included in a housing (not shown) that is formed in (or attached to) the outside of the main frame of the wearable device 300. Each "module" herein may comprise circuitry.

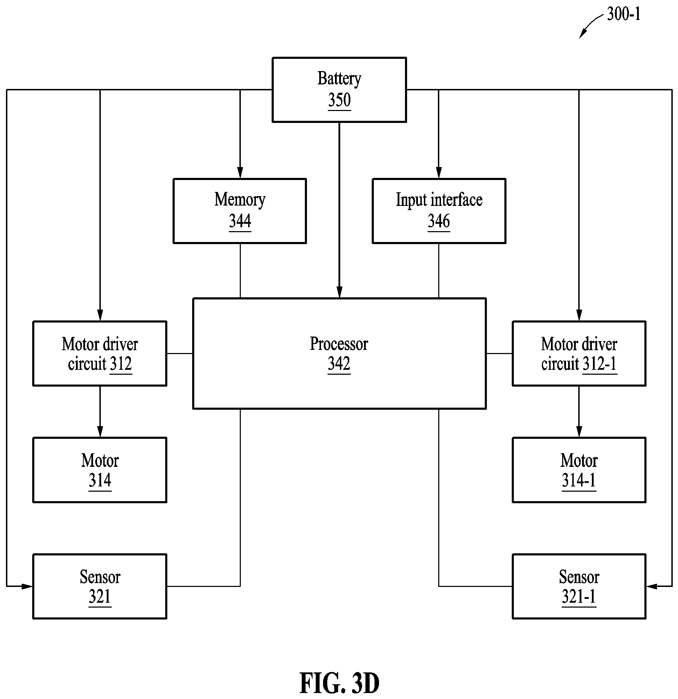

The driver 310 may include a motor 314 and a motor driver circuit 312 for driving the motor 314. The sensor 320 may include at least one sensor 321. The controller 340 may include a processor 342, a memory 344, and an input interface 346. Although only one sensor 321, one motor driver circuit 312, and one motor 314 is illustrated in FIG. 3c, this is provided merely as an example and a wearable device (e.g., a wearable device 300-1) may include a plurality of sensors 321 and 321-1, a plurality of motor driver circuits 312 and 312-1, and a plurality of motors 314 and 314-1 according to another example as illustrated in FIG. 3d. Also, according to implementation, the wearable device 300 may include a plurality of processors. The number of motor driver circuits, the number of motors, or the number of processors may vary according to a body part on which the wearable device 300 is worn.

The following descriptions of the sensor 321, the motor driver circuit 312, and the motor 314 may also be applicable to the sensor 321-1, the motor driver circuit 312-1, and the motor 314-1 illustrated in FIG. 3d.

The driver 310 may drive a hip joint of the user. For example, the driver 310 may be disposed on a right hip portion of the user and/or a left hip portion of the user. The driver 310 may be additionally disposed on knee portions and ankle portions of the user. The driver 310 may include the motor 314 configured to generate a rotational torque and the motor driver circuit 312 configured to drive the motor 314.

The sensor 320 may measure an angle of the hip joint of the user when the user walks. Information on the hip joint angle sensed by the sensor 320 may include a right hip joint angle, a left hip joint angle, a difference between both hip joint angles, and a hip joint motion direction. For example, the sensor 321 may be disposed in the driver 310. According to a position of the sensor, the sensor 320 (e.g., 321 and/or 321-1) may additionally measure a knee angle and an ankle angle of the user. The sensor 321 may include an encoder. The information on the hip joint angle measured by the sensor 320 may be transmitted to the controller 340.

According to an example embodiment, the sensor 320 may include a potentiometer. The potentiometer may sense an R-axis joint angle and an L-axis joint angle, and an R-axis joint angular velocity and an L-axis angular velocity, based on a walking motion of the user. In this case, R and L axes may be reference axes for a right leg and a left leg of the user, respectively. For example, the R and L axes may be set to be vertical to the ground and set such that a front side of a body of a person has a negative value and a rear side of the body has a positive value.

The IMU 330 may measure acceleration information and pose information when the user walks. For example, the IMU 330 may sense an X-axis acceleration, a Y-axis acceleration, and a Z-axis acceleration, and an X-axis angular velocity, a Y-axis angular velocity, and a Z-axis angular velocity, based on a walking motion of the user. The acceleration information and the pose information measured by the IMU 330 may be transmitted to the controller 340.

The wearable device 300 may detect a point at which the foot of the user lands based on the acceleration information measured by the IMU 330.

A pressure sensor (not shown) may be disposed on the sole of the foot of the user to detect a landing time of the foot of the user.

In addition to the sensor 320 and the IMU 330 described above, the wearable device 300 may include other sensors (e.g., an electromyogram (EMG) sensor) configured to sense a change in a quantity of motion of the user or a change in biosignal based on a walking motion of the user.

The controller 340 may control an overall operation of the wearable device 300. For example, the controller 340 may receive information sensed by each of the sensor 320 and the IMU 330. The information sensed by the IMU 330 may include the acceleration information and the pose information, and the information sensed by the sensor 320 may include information on the right hip joint angle, the left hip joint angle, the difference between both hip joint angles, and the hip joint motion direction. According to embodiments, the controller 340 may calculate the difference between both hip joint angles based on the right hip joint angle and the left hip joint angle. The controller 340 may generate a signal for controlling the driver 310 based on the sensed information. For example, the generated signal may be an assistance force for assisting the user in walking. In another example, the generated signal may be a resistance force for hindering the walking of the user. The resistance force may be provided to the user to assist the user in doing an exercise.

According to an example embodiment, the processor 342 of the controller 340 may control the driver 310 to provide the resistance force to the user.

For example, the driver 310 may provide the resistance force to the user by applying an active force to the user through the motor 314. In another example, the driver 310 may provide the resistance force to the user using back-drivability of the motor 314 without applying an active force to the user. Here, the back-drivability of a motor may indicate reactivity of a rotation axis of the motor in response to an external force, and a greater back-drivability may indicate that the motor may more readily respond to an external force acting on the rotation axis, that is, the motor may more readily rotate around the rotation axis. For example, even when the same external force is applied to the rotation axis of the motor, a degree of rotation of the motor around the rotation axis may change according to a degree of the back-drivability.

In another example, the driver 310 may provide the resistance force to the user by outputting a torque in a direction that hinders a movement of the user.

According to an example embodiment, the processor 342 of the controller 240 may control the driver 310 such that the driver 310 outputs a torque (or an assistance torque) to assist the user in walking. For example, in the hip-type wearable device 300, the driver 310 may be disposed on each of a left hip portion and a right hip portion, and the controller 340 may output a control signal for controlling the driver 310 to generate a torque.

The driver 310 may generate a torque based on the control signal output by the controller 340. A torque value for generating the torque may be externally set or be set by the controller 340. For example, to indicate a magnitude of the torque value, the controller 340 may use a magnitude of a current for a signal transmitted to the driver 310. That is, as the magnitude of the current received by the driver 310 increases, the torque value may increase. In another example, the processor 342 of the controller 340 may transmit the control signal to the motor driver circuit 312 of the driver 310, and the motor driver circuit 312 may generate a current corresponding to the control signal to control the motor 314.

The battery 350 may supply power to the components of the wearable device 300. The wearable device 300 may further include a circuit (e.g., a power management integrated circuit (PMIC)) configured to convert power of the battery 350 to match an operating voltage of the components of the wearable device 300 and provide the power to the components of the wearable device 300. In addition, the battery 350 may or may not supply power to the motor 314 based on an operation mode of the wearable device 300.

The communication module 352, comprising communication circuitry, may support the establishment of a direct (e.g., wired) communication channel or a wireless communication channel between the wearable electronic device 300 and an external electronic device, and support the communication through the established communication channel. The communication module 352 may include one or more communication processor configured to support direct (e.g., wired) communication or wireless communication. According to an example embodiment, the communication module 352 may include a wireless communication module (e.g., a cellular communication module, a short-range wireless communication module, or a GNSS communication module) or a wired communication module (e.g., an LAN communication module or a PLC module). A corresponding one of these communication modules may communicate with the external electronic device via a first network (e.g., a short-range communication network such as Bluetooth^{™}, Wi-Fi direct, or IrDA) or a second network (e.g., a legacy cellular network, a 5G network, a next-generation communication network, the Internet, or a computer network). These various types of communication modules may be implemented as a single component (e.g., a single chip), or may be implemented as multiple components (e.g., multiple chips) separate from each other. Each processor herein comprises processing circuitry.

FIG. 4 is a diagram illustrating a wearable device communicating with an electronic device according to an example embodiment.

Referring to FIG. 4, the wearable device 300 may communicate with an electronic device 201. For example, the electronic device 201 may be an electronic device of a user of the wearable device 300. According to an example embodiment, the wearable device 300 and the electronic device 201 may be connected using short-range wireless communication.

The electronic device 201 may display a user interface (UI) for controlling an operation of the wearable device 300 on a display 201-1. The UI may include, for example, at least one soft key through which the user may control the wearable device 300.

The user may input a command for controlling the operation of the wearable device 300 through the UI on the display 201-1 of the electronic device 201, and a server 1400 (e.g., see Fig. 14) may generate a control command corresponding to the command and transmit the generated control command to the wearable device 300. The wearable device 300 may operate according to the received control command, and transmit a control result to the electronic device 201. The electronic device 201 may display a control completion message on the display 201-1 of the electronic device 201.

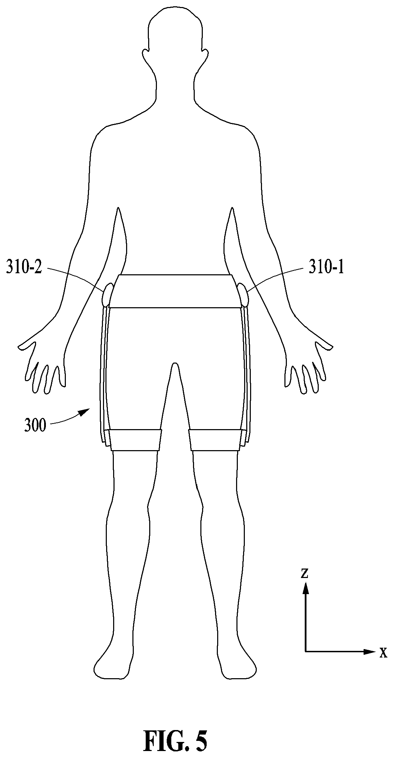

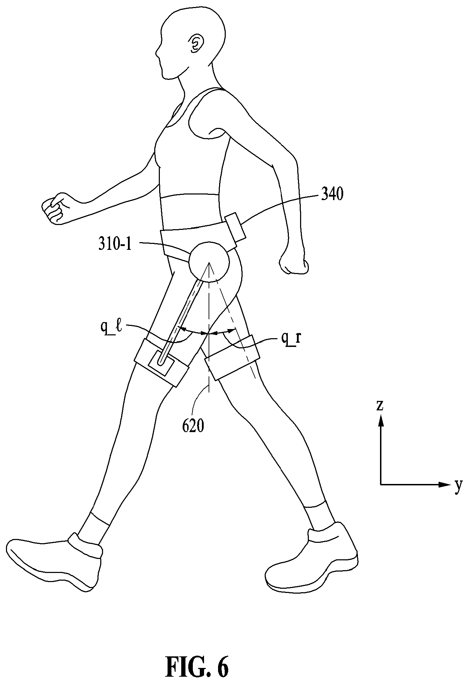

FIGS. 5 and 6 are diagrams illustrating a method of outputting a torque by a wearable device according to embodiments.

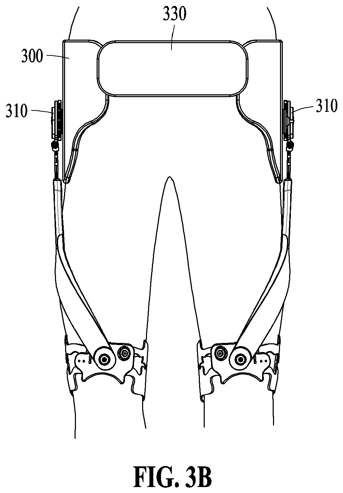

Referring to FIGS. 5 and 6, the drivers 310-1 and 310-2 of the wearable device 300 of FIG. 3 may be disposed at or near a hip joint of a user, and the controller 340 of the wearable device 300 may be disposed at or near a waist of the user. However, the positions of the drivers 310-1 and 310-2 and the controller 340 are not limited to the example positions illustrated in FIGS. 5 and 6.

The wearable device 300 may measure (or sense) a left hip joint angle q_l and a right hip joint angle q_r of the user. For example, the wearable device 300 may measure the left hip joint angle q_l of the user through a left encoder, and measure the right hip joint angle q_r of the user through a right encoder. As illustrated in FIG. 6, the left hip angle q_l may be a negative value because a left leg of the user is in front of a reference line 620, and the right hip angle q_r may be a positive value because a right leg of the user is behind the reference line 620. According to implementation, the right hip joint angle q_r may be negative when the right leg is in front of the reference line 620, and the left hip joint angle q_l may be positive when the left leg is behind the reference line 620.

According to an example embodiment, the wearable device 300 may acquire a first angle (e.g., q_r) and a second angle (e.g., q_l) by filtering a first raw angle (e.g., q_r_raw) of a first joint (e.g., the right hip joint) and a second raw angle (e.g., q_l raw) of a second joint (e.g., the left hip joint) measured by the sensor 320. For example, the wearable device 300 may filter a first primitive angle and a second primitive angle based on a first previous angle and a second previous angle measured with respect to a previous time.

According to an example embodiment, the wearable device 300 may determine a torque value τ(t) based on the left hip joint angle q_l, the right hip joint angle q_r, an offset angle c, a sensitivity α, a gain κ, and a delay Δt, and control the motor driver circuit 312 of the wearable device 300 to output the determined torque value τ(t). A force provided to the user by the torque value τ(t) may be referred to as force feedback. For example, the wearable device 300 may determine the torque value τ(t) based on Equation 1 below. [Equation 1] $\begin{matrix}y = sin \left(q_r\right) - sin \left(q_l\right) \\ τ \left(t\right) = κy \left(t-Δt\right)\end{matrix}$

In Equation 1 above, y may denote a state factor, q_r may denote a right hip joint angle, and q_l may denote a left hip joint angle. According to Equation 1, the state factor y may be associated with a distance between both legs. For example, y being 0 may indicate a state (e.g., a crossing state) in which the distance between the legs is 0, and an absolute value of y being maximum may indicate a state (e.g., a landing state) in which an angle between the legs is maximum. When q_r and q_l are measured at a time t, the state factor may be represented as y(t).

The gain κ is a parameter indicating a magnitude and direction of an output torque. As the magnitude of the gain κ increases, a greater torque may be output. When the gain κ is a negative value, a torque acting as a resistance force may be output to the user. When the gain κ is a positive value, a torque acting as an assistance force may be output to the user. The delay Δt may be a parameter associated with a torque output timing. The value of the gain *κ* and the value of the delay Δt may be preset, and may be adjustable by the user or the wearable device 300. A model for outputting a torque acting as an assistance force to a user based on parameters such as Equation 1, the gain κ, and the delay Δt may be a torque output model (or a torque output algorithm). The wearable device 300 may determine a torque magnitude and a torque delay to be output by inputting values of input parameters received through sensors to the torque output model.

According to an example embodiment, the wearable device 300 may determine a first torque value by applying a first gain value and a first delay value as parameter values determined with respect to the state factor y(t) to a first state factor y(t), based on Equation 2 below. [Equation 2] $\begin{matrix}{τ}_{l} \left(t\right) = κy \left(t-Δt\right) \\ {τ}_{r} \left(t\right) = - κy \left(t-Δt\right)\end{matrix}$

The calculated first torque value may include a value related to the first joint and a value related to the second joint since the first torque value is applied to both legs. For example, *τₗ*(*t*) may be a value related to the left hip joint which is the second joint, and *τᵣ*(*t*) may be a value related to the right hip joint, which is the first joint. *τₗ*(*t*) and *τᵣ*(*t*) may have the same magnitude and opposite torque directions. The wearable device 300 may control the motor driver 312 of the wearable device 300 to output a torque corresponding to the first torque value.

According to an example embodiment, when the user performs a gait in which the left leg and the right leg are asymmetrical, the wearable device 300 may provide an asymmetrical torque to both legs of the user to assist the asymmetric gait. For example, the wearable device 300 may provide a stronger assistance force to the leg with a shorter step length or slower swing speed. Hereinafter, a leg with a shorter step length or a slower swing speed may be referred to as an affected leg or a target leg.

In general, the swing time or the step length of an affected leg may be shorter than the swing time or the step length of an unaffected leg. According to an example embodiment, to assist a gait of the user, a method of adjusting the timing of a torque acting on an affected leg may be considered. For example, an offset angle may be added to an actual joint angle for the affected leg to increase the output time of the torque for assisting the swing motion of the affected leg. c may be a value of a parameter indicating an offset angle between joint angles. By adding the offset angle to the actual joint angle of the affected leg, the value of an input parameter input to the torque output model mounted (or applied) to the wearable device 300 may be adjusted. For example, the values of q_r and q_l may be adjusted through Equation 3 below. cᵣ may denote an offset angle with respect to the right hip joint, and c₁ may denote an offset angle with respect to the left hip joint. [Equation 3] $\begin{matrix}{q}_{_ r} \left(t\right) ← {q}_{_ r} \left(t\right) + {c}_{r} \\ {q}_{_ l} \left(t\right) ← {q}_{_ l} \left(t\right) + {c}_{l}\end{matrix}$

According to an example embodiment, the wearable device 300 may filter the state factor to reduce discomfort of the user due to an irregular torque output. For example, the wearable device 300 may determine an initial state factor y _{raw} (t) of the current time t based on the first angle of the first joint and the second angle of the second joint, and determine the first state factor y(t) based on a previous state factor y ^{prv} determined with respect to a previous time t-1 and the initial state factor y _{raw} (t). The current time t may indicate a processing time for t-th data (or sample), and the previous time t-1 may indicate a processing time for t-1th data. For example, a difference between the current time t and the previous time t-1 may be an operation cycle of a processor configured to generate or process corresponding data. The sensitivity α may be a value of a parameter indicating sensitivity. For example, the sensitivity value may be continuously adjusted during a test walk, but to reduce the complexity of calculation, the sensitivity value may be preset to a predetermined value.

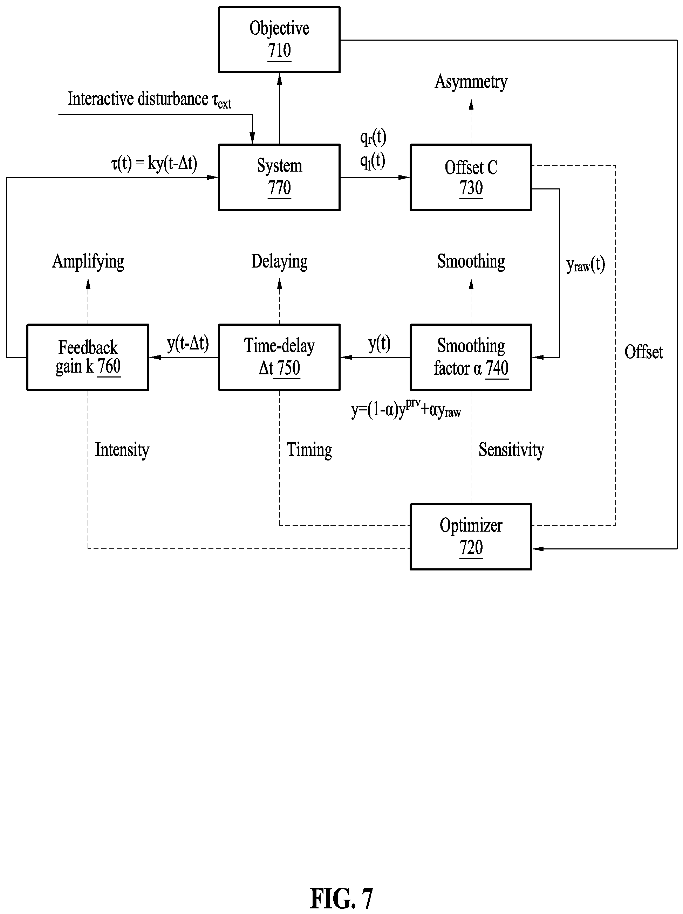

FIG. 7 is a block diagram for optimizing parameter values of a wearable device according to an example embodiment.

According to an example embodiment, the electronic device 201 may optimize values of control parameters used by a system 770 (e.g., the wearable device 300 of FIG. 3) to output a torque. For example, optimized values of the control parameters may be determined by an optimizer 720 of the electronic device 201. According to an example embodiment, the control parameters may include at least one of an offset angle 730, a sensitivity 740, a delay 750, or a gain 760 or combination thereof.

According to an example embodiment, the optimizer 720 may optimize the values of the control parameters based on test sensing information acquired through testing a gait of a user of the wearable device 300. For example, a gait test may be for checking the current state of the gait of the user, and the wearable device 300 may not provide an assistance force or a resistance force to the user while the gait test is being performed. The optimizer 720 may optimize the values of the control parameters based on the test sensing information and an exercise objective 710 preset by the user through the gait test.

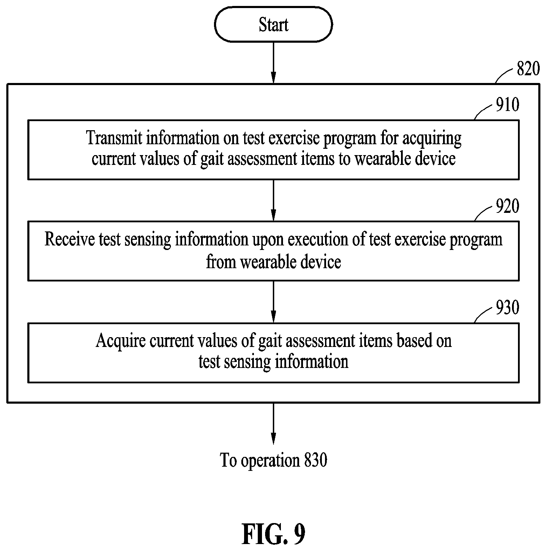

The determining of optimal values of control parameters through a gait test by the electronic device 201 will be described in detail below with reference to FIG. 9.

FIG. 8 is a flowchart illustrating a method of providing an exercise program to a user according to an example embodiment.

According to an example embodiment, operations 810 to 880 may be performed by an electronic device (e.g., the electronic device 110 of FIG. 1 or the electronic device 201 of FIG. 2).

In operation 810, the electronic device may receive target exercise information from a user. For example, the target exercise information may include target exercise time information and target exercise section information.

According to an example embodiment, the target exercise time information may be information on an entire exercise time (e.g., 30 minutes or 1 hour) during which the user currently wants to perform exercise.

According to an example embodiment, the target exercise section information may include an exercise route. For example, the exercise route may include information on a start point, an end point, and detailed routes between the start point and the end point.

In operation 820, the electronic device may acquire current values of gait assessment items. For example, the gait assessment items may include at least one of a step length, a gait speed, a gait symmetry, or a gait rhythm or combination thereof.

According to an example embodiment, the electronic device may acquire the current values of the gait assessment items based on previous sensing information acquired while performing a previous exercise program using a wearable device (e.g., the wearable device 120 of FIG. 1 or the wearable device 300 of FIG. 3a). For example, the previous exercise program may be the most recently performed exercise program or accumulated exercise programs.

According to an example embodiment, the electronic device may cause a user to perform a test exercise program through a wearable device, and acquire current values of gait assessment items based on test sensing information acquired as a result of the execution of the test exercise program. The acquiring of current values of gait assessment items based on a test exercise program will be described in detail below with reference to FIG. 9.

According to an example embodiment, a step length may be a distance between the left foot and the right foot when walking. For example, when the left leg and the right leg touch the ground at the same time, the legs and the ground may form a triangle. In this example, the step length representing the remaining sides of the triangle may be calculated based on a left hip joint angle, a right hip joint angle, and the leg length. For example, an average step length for a plurality of steps performed by the user may be acquired as the step length.

According to an example embodiment, the gait speed may be acquired based on a moving distance and a moving time. For example, an IMU may be used to estimate the gait speed. The IMU may acquire acceleration of 3 degrees of freedom and a rotation rate of 3 degrees of freedom. The electronic device may acquire the gait speed by inputting the acquired acceleration of 3 degrees of freedom and rotation rate of 3 degrees of freedom to a pre-trained gait speed estimation model based on a long short-term memory (LSTM). For example, the gait speed estimation model may be pre-trained through machine learning. For example, the gait speed estimation model may be a model using dynamic analysis. For example, the gait speed estimation model may be a model using a method of extracting features and performing linear regression.

According to an example embodiment, the gait rhythm may indicate how consistently the user walks. For example, the gait rhythm may be calculated by Equation 4. [Equation 4] $100 \times \frac{Stride Time Standard Deviation}{Stride Time Mean}$

In Equation 4, the stride time standard deviation may indicate the standard deviation of a stride time, and the stride time mean may indicate an average stride time. That is, the gait rhythm may be a coefficient of variation (CV) with respect to the stride time.

According to an example embodiment, the stride time may be calculated based on a movement of periodically moving legs. For example, a step time of the left leg may be calculated based on a time between time points when a sign of an angular velocity of the left hip joint changes, and a step time of the right leg may be calculated based on a time between time points when a sign of an angular velocity of the right hip joint changes. For example, to reduce noise, 50 angular velocity samples may be averaged, and the step time and the stride time of each leg may be calculated based on a time between time points when a sign of the mean value changes. For example, one stride time may be calculated as the sum of the step time of the left leg and the step time of the right leg.

According to an example embodiment, the gait symmetry may indicate how symmetrical the gait of the left leg and the gait of the right leg are. For example, the gait symmetry may be calculated based on a ratio of a step time of one leg to a step time of the other leg. For example, the gait symmetry may be calculated by Equation 5. [Equation 5] $100 \times \frac{\left|Left Step Time Mean-Right Step Time Mean\right|}{0.5 ⋅ \left(Left Step Time Mean+Right Step Time Mean\right)}$

In Equation 5, the left step time mean may indicate an average time with respect to the steps of the left leg, and the right step time mean may indicate an average time with respect to the steps of the right leg. That is, the gait symmetry may be a symmetry index (SI) between steps. For example, the gait symmetry calculated as the SI may indicate temporal gait symmetry, and when time is converted into a length corresponding to time, the gait symmetry may indicate spatial gait symmetry.

In operation 830, the electronic device may determine optimal values of the control parameters that may satisfy target values of the gait assessment items based on the target exercise information and the current values of the gait assessment items.

According to an example embodiment, the electronic device may set target values of gait assessment items based on a target exercise objective selected by a user from among a plurality of exercise objectives. For example, the plurality of exercise objectives may include two or more of improving gait ability, improving gait posture, improving cardiovascular health, and improving muscular strength. For example, the target values of the assessment items may be adjusted in detail based on body information, age, and gender of the user. The electronic device may prioritize or sort the assessment items that are preferentially required for the user among the assessment items based on the target exercise objective.

According to an example embodiment, the electronic device may determine a target assessment item to be improved based on current values of gait assessment items, and determine one or more control parameter that has the greatest effect on a value of the target assessment item. For example, when the target assessment item is improvement of step length, timing and a gain may be determined as control parameters. For example, when the target assessment item is improvement of gait speed, sensitivity may be determined as the control parameter. For example, when the target assessment item is improvement of gait symmetry, an offset angle may be determined as the control parameter. For example, when the target assessment item is improvement of gait rhythm, sensitivity may be determined as the control parameter. Values of other control parameters that are not determined with respect to the target assessment item may be fixed during the optimization process. According to an example embodiment, when a correlation between the target assessment item and the control parameters is established in advance through a look-up table or a regression model, a process for determining an optimal value of the control parameter may be performed quickly.

According to an example embodiment, the electronic device may determine the optimal value of the control parameter such that the target values of the gait assessment items may be satisfied within a range that satisfies the set objective function. For example, the objective function may be expressed as Equation 6 below. 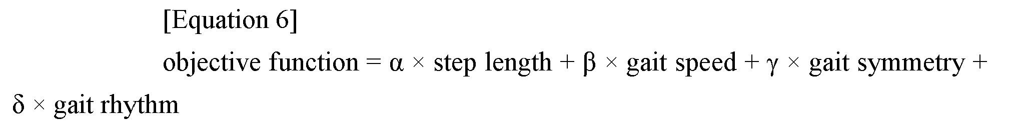[Equation 6] objective function = α × step length + β × gait speed + γ × gait symmetry + δ × gait rhythm

In Equation 6, α, β, γ, and δ may denote weights for each assessment item. The value of the objective function may be used as an indicator of directionality, and for example, an optimal value of the control parameter may be determined such that a value improved over a previous value of the objective function is calculated. For example, when the user exercises daily, a value of today's control parameter may be determined as the optimal value so that a value improved over a value of the objective function by yesterday's exercise is calculated.

According to an example embodiment, the electronic device may further consider target exercise information to determine an optimal value of the control parameter. For example, as a target exercise time becomes shorter or a target exercise distance increases, the torque output timing may be shortened as the gait speed increases. In addition, when it is determined that it is difficult to achieve the target exercise distance within the target exercise time only by adjusting the torque output timing based on the gait ability of the current user, the electronic device may determine a gain value such that the magnitude of a torque output for assisting the user in walking increases. Contrary to the above embodiment, when it is determined that the target exercise distance can be achieved within the target exercise time based on the gait ability of the current user, the electronic device may determine a gain value such that a torque is output to the user that hinders the gait of the user. The magnitude of the torque that interferes with the gait may vary depending on the current gait ability of the user. For example, the current gait ability of the user may correspond to any one of an upper group, a middle group, or a lower group.

According to an example embodiment, the electronic device may further consider topographic information on an exercise route to determine an optimal value of the control parameter. The electronic device may acquire topographic information on the exercise route received from the user. For example, the topographic information on the exercise route may be acquired based on a database in the electronic device. For example, the topographic information on the exercise route may be acquired from an external server that provides a map service. According to an example embodiment, when the terrain of the exercise route is uphill, the electronic device may determine a gain value so that a torque for assisting walking is output to the user.

According to an example embodiment, the optimal value of the control parameter may be determined as any one predetermined value, or may be determined as a range including the predetermined value. The optimal values of a plurality of control parameters may be referred to as an optimal value set.

In operation 840, the electronic device may acquire one or more recommended exercise program based on an optimal value (or an optimal value set) of the control parameter.

According to an example embodiment, values of control parameters used by an exercise program or control parameters applied to the exercise program may be matched with each exercise program. An intensity and output timing of an assistance force or a resistance force output through the exercise program may vary depending on a value of the control parameter applied to the exercise program. For example, the exercise program may provide the user with the same assistance force or resistance force within an entire exercise time through a wearable device. For example, the exercise program may divide the entire exercise time into a plurality of sections, and provide different assistance or resistance forces to the user through the wearable device in the plurality of sections.

According to an example embodiment, the electronic device may determine candidate exercise programs corresponding to the optimal value of the control parameter among a plurality of exercise programs stored in the electronic device. For example, the electronic device may determine the candidate exercise programs corresponding to the optimal value of the control parameter based on a database in which the values of the control parameters applied to each of the plurality of exercise programs are stored.

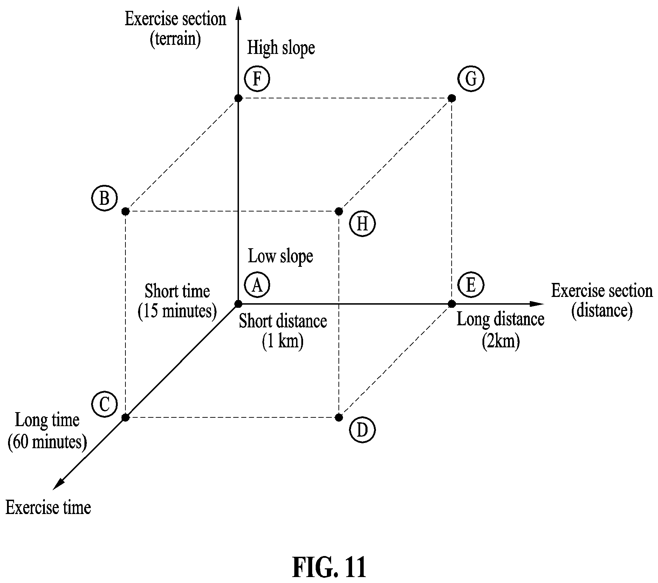

According to an example embodiment, the electronic device may determine recommended exercise programs based on a preset filtering condition among the candidate exercise programs. For example, the filtering condition may be preset by the user. For example, the filtering condition may be set based on a history of an exercise program recently performed by the user. Even when the user frequently exercises in the same exercise environment, the recommended exercise program may vary according to the exercise history, so to the user it may feel like exercising in a different exercise environment than the corresponding exercise environment. The determining of recommended exercise programs and candidate exercise programs will be described in detail below with reference to FIG. 11.

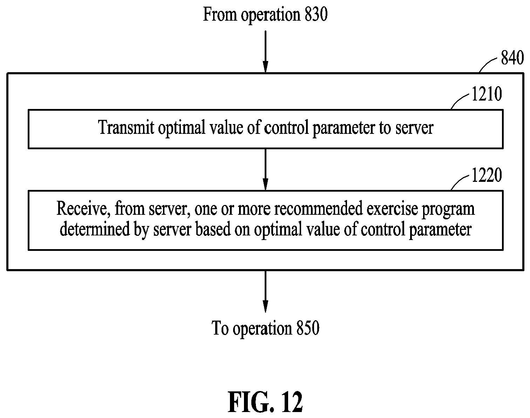

According to an example embodiment, the electronic device may transmit an optimal value of a control parameter to a server (e.g., the server 140 of FIG. 1) and receive recommended exercise programs from the server. The acquiring of a recommended exercise program through the server performed by the electronic device will be described in detail below with reference to FIG. 12.

In operation 850, the electronic device may determine a target exercise program among one or more recommended exercise program. For example, the electronic device may determine the target exercise program by receiving a selection of the target exercise program from the user.

According to an example embodiment, the electronic device may output one or more recommended exercise program through a display of the electronic device or a display of an additional device (e.g., the additional device 130 of FIG. 1) connected to the electronic device, and receive a selection of a target exercise program from the user through a touch input or a button input.

According to an example embodiment, the electronic device may provide the user with a gait age achieved when a recommended exercise program is performed. For example, an average age of users who can normally perform the recommended exercise program without the help of the wearable device may be matched with the recommended exercise program in advance.

In operation 860, the electronic device may transmit information on the target exercise program to the wearable device worn by the user. For example, the information on the target exercise program may include information on the values of the control parameters applied to the target exercise program. While the user performs a target exercise program while wearing the wearable device, the wearable device may provide an assistance force or a resistance force to the body (e.g., legs) of the user based on the received values of the control parameters. For example, the value of the control parameter may represent a trajectory for the target exercise time.

For example, the target exercise program may provide the user with the same assistance force or resistance force for the entire exercise time. As another example, the target exercise program may divide the entire exercise time into a plurality of sections, and provide different assistance or resistance forces to the user in the plurality of sections. As another example, the target exercise program may divide the entire exercise route into a plurality of detailed exercise routes, and provide different assistance or resistance forces to the user in the plurality of detailed exercise routes.

In operation 870, the electronic device may receive sensing information from the wearable device while the target exercise program is being performed by the wearable device. For example, the wearable device may generate sensing information on a gait of the user using at least one sensor and transmit the sensing information to the electronic device.

For example, the sensing information may include an angle of a left hip joint and an angle of a right hip joint of the user generated by the encoder. For example, the sensing information may include acceleration information and pose information generated by an IMU. For example, the sensing information may include biometric information generated by a biosensor. For example, the sensing information may include position information generated by a global positioning system (GPS) sensor. Each element in the sensing information may be associated with a timestamp.

According to an example embodiment, the electronic device may receive additional sensing information from an additional device connected to the electronic device. For example, when the additional device is a smartwatch (e.g., the smartwatch 132 of FIG. 1), the electronic device may receive a heart rate from the smartwatch as additional sensing information.

In operation 880, the electronic device may provide feedback information on the execution of the target exercise program to the user based on the sensing information.

According to an example embodiment, the electronic device may determine whether target values of gait assessment items are satisfied based on the sensing information, and provide different feedback information to the user based on whether the target values are satisfied. The providing of feedback information to the user will be described in detail below with reference to FIG. 13.

According to an example embodiment, the electronic device may provide feedback information to the user through a user interface device of the wearable device. For example, the feedback information may be provided to the user through a speaker, a display, or a haptic device of the wearable device.

According to an example embodiment, the electronic device may provide feedback information to the user through an additional device (e.g., the additional device 130 of FIG. 1) connected, directly or indirectly, to the electronic device. For example, when the additional device is a wireless earphone (e.g., the wireless earphone 131 of FIG. 1), auditory feedback information may be provided to the user through the wireless earphone. For example, when the additional device is a smartwatch (e.g., the smartwatch 132 of FIG. 1) or smart glasses (e.g., the smart glasses 133 of FIG. 1), visual, auditory, or tactile feedback information may be provided to the user.

According to an example embodiment, the user may identify a current state of a gait through the feedback information, and change how an exercise is performed based on the identified current state of the gait. For example, when the feedback information indicates that the gait speed is low, the user may increase the gait speed.

According to an example embodiment, after the user has finished the exercise for a target exercise program, the electronic device may evaluate a performance result of the exercise, and store the performance result and the assessment. For example, the performance result and the assessment for the current exercise may be used as current values of gait assessment items of a following exercise.

FIG. 9 is a flowchart illustrating a method of acquiring current values of gait assessment items according to an example embodiment.

According to an example embodiment, operation 820 described above with reference to FIG. 8 may include operations 910 to 930 to be described hereinafter with reference to FIG. 9.

In operation 910, an electronic device may transmit information on a test exercise program for acquiring current values of gait assessment items to a wearable device.

According to an example embodiment, the test exercise program may be an exercise program for checking a current state of a gait of a user. For example, the test exercise program may instruct the user to start walking, and the user may walk for a preset distance (e.g., 10 meters) or a preset time (e.g., 5 seconds). While the user performs the test walk, the test exercise program may not provide an assistance force or a resistance force to the user through the wearable device.

In operation 920, the electronic device may receive test sensing information on the execution of the test exercise program from the wearable device. For example, the wearable device may generate the test sensing information on the test walk of the user using at least one sensor, and transmit the test sensing information to the electronic device.

For example, the test sensing information may include an angle of a left hip joint and an angle of a right hip joint of the user generated by an encoder. For example, the test sensing information may include acceleration information and pose information generated by an IMU. For example, the test sensing information may include biometric information generated by a biosensor. Each element in the test sensing information may be associated with a timestamp.

In operation 930, the electronic device may acquire current values of gait assessment items based on the test sensing information. For example, the gait assessment items may include at least one of a step length, gait speed, gait symmetry, or gait rhythm or combination thereof. A further description of the acquiring of the current values for each of a step length, gait speed, gait symmetry, and gait rhythm may be replaced with the description of operation 820 described above with reference to FIG. 8.

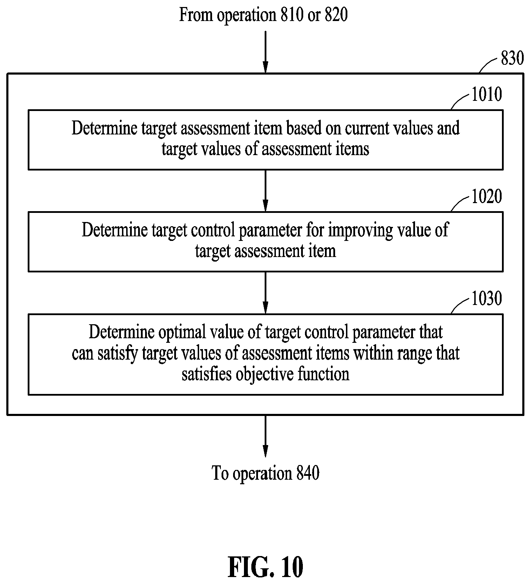

FIG. 10 is a flowchart illustrating a method of determining an optimal value of a control parameter according to an example embodiment.

According to an example embodiment, operation 830 described above with reference to FIG. 8 may include operations 1010 to 1030 to be described hereinafter with reference to FIG. 10.

In operation 1010, an electronic device may determine a target assessment item based on current values and target values of gait assessment items. For example, assessment items may be arranged in an order of a greatest difference between the current value and the target value of each assessment item, and one or more target assessment item may be determined based on the arrangement.

In operation 1020, the electronic device may determine a target control parameter for improving a value of the target assessment item. For example, when the target assessment item is improvement of step length, timing and a gain may be determined as the control parameter. For example, when the target assessment item is improvement of gait speed, sensitivity may be determined as the control parameter. For example, when the target assessment item is improvement of gait symmetry, an offset angle may be determined as the control parameter. For example, when the target assessment item is improvement of gait rhythm, sensitivity may be determined as the control parameter. Values of other control parameters that are not determined with respect to the target assessment item may be fixed during the optimization process.

In operation 1030, the electronic device may determine an optimal value of a target control parameter that may satisfy target values of assessment items within a range that satisfies the objective function. For example, the objective function may be expressed by the above-mentioned Equation 6.

According to an example embodiment, to determine the optimal value of the target control parameter, target exercise time information, target exercise section information, current values of assessment items, and target values of assessment items may be considered. For example, the optimal value of the target control parameter may be determined such that an exercise objective of the user is achieved while satisfying both the target exercise time and the target exercise section. For example, the objective function may be satisfied by the achievement of the exercise objective of the user, and the objective function may be satisfied when a value of the objective function related to a current exercise becomes greater than a value of the objective function related to a previous exercise.

FIG. 11 is a diagram illustrating a method of determining recommended exercise programs based on target exercise information according to an example embodiment.

The three-dimensional space shown in FIG. 11 illustrates an exercise environment expressed by an exercise time, an exercise distance, and an exercise terrain, according to an example. As a value of each axis increases, a greater exercise intensity may be required from the user.

According to an example embodiment, an electronic device or a server receiving an optimal value (or an optimal value set) of a control parameter from the electronic device may determine recommended exercise programs based on the optimal value of the control parameter.

For example, when an exercise section with a record of previously performed exercise is selected, but a shorter exercise time than before is selected (e.g., moving from point C to point A), an exercise program that increases a gait speed compared to the previously performed exercise program may be determined as the recommended exercise program.

For example, when the same exercise time as a previous exercise time is selected, but a shorter exercise distance is selected (e.g., moving from point E to point A), an exercise program that increases an exercise load (e.g., a resistance force) compared to the previously performed exercise program may be determined as the recommended exercise program.

For example, when a same exercise time as a previous exercise time is selected, but an easier exercise terrain is selected (e.g., moving from point F to point A), an exercise program that increases an exercise load (e.g., a resistance force) compared to the previously performed exercise program may be determined as the recommended exercise program.

For example, when the same exercise time as a previous exercise time is selected, but a longer exercise distance is selected (e.g., moving from point A to point E), an exercise program that decreases an exercise load (e.g., a resistance force) or provides an assistance force compared to the previously performed exercise program may be determined as the recommended exercise program.

For example, when the same exercise time as a previous exercise time is selected, but a more difficult exercise terrain is selected (e.g., moving from point A to point F), an exercise program that decreases an exercise load (e.g., a resistance force) or provides an assistance force compared to the previously performed exercise program may be determined as the recommended exercise program.

For example, when an exercise time, exercise distance, and exercise terrain different from a previous exercise time, exercise distance and exercise terrain are selected (e.g., moving from point D to point F), various recommended exercise programs may be determined based on the optimal value of the control parameter.

According to an example embodiment, when a plurality of recommended exercise programs are determined, the server may preferentially recommend an exercise program highly suited to the user's preferences based on exercise programs performed by users other than the user of the electronic device. The server may include a database in which information related to exercise performed by users using the wearable device is stored. For example, based on the target exercise section information, exercise programs performed by other users in the target exercise section may be acquired. For example, other users to be referenced may have the same exercise objective as the user or have the same gait age as the user.

FIG. 12 is a flowchart illustrating a method of acquiring recommended exercise programs through a server according to an example embodiment.

According to an example embodiment, operation 840 described above with reference to FIG. 8 may include operations 1210 and 1220 to be described hereinafter with reference to FIG. 12.

In operation 1210, an electronic device (e.g., the electronic device 110 of FIG. 1 or the electronic device 201 of FIG. 2) may transmit an optimal value (or an optimal value set) of a control parameter to a server (e.g., the server 140 of FIG. 1).

According to an example embodiment, the server may determine one or more recommended exercise program based on the optimal value of the control parameter.

In operation 1220, the electronic device may receive, from the server, one or more recommended exercise program determined by the server based on the optimal value of the control parameter.

FIG. 13 is a flowchart illustrating a method of providing feedback information to a user according to an example embodiment.

According to an example embodiment, operation 880 described above with reference to FIG. 8 may include operations 1310 to 1330 to be described hereinafter with reference to FIG. 13.

In operation 1310, an electronic device may determine whether target values of gait assessment items are being satisfied based on sensing information received from a wearable device.

According to an example embodiment, the electronic device may calculate current values of gait assessment items based on the sensing information, and may determine whether the calculated current values correspond to the target values. For example, the current values for a step length, gait speed, gait symmetry, and gait rhythm may be calculated, and it may be determined whether the current values correspond to the target values. When a user is performing well on a target exercise program, the current values of the assessment items may correspond to the target values, and when the user is not performing well on the target exercise program, the current value of at least one assessment item may not reach the target value.

In operation 1320, when the target values of the assessment items are not satisfied, the electronic device may determine a requested action for satisfying the target values. For example, when a target value of a gait speed is not satisfied, 'walk faster than the current speed' may be determined as the requested action to satisfy the target value of the gait speed. For example, when a target value of a step length is not satisfied, 'widen your step length' may be determined as the requested action to satisfy the target value of the step length.

In operation 1330, the electronic device may provide feedback information including the requested action to the user.

According to an example embodiment, the electronic device may provide feedback information to the user through a user interface device of the wearable device. For example, the feedback information may be provided to the user through a speaker, a display, or a haptic device of the wearable device.

According to an example embodiment, the electronic device may provide feedback information to the user through an additional device (e.g., the additional device 130 of FIG. 1) connected to the electronic device. For example, when the additional device is a wireless earphone (e.g., the wireless earphone 131 of FIG. 1), auditory feedback information may be provided to the user through the wireless earphone. For example, when the additional device is a smartwatch (e.g., the smartwatch 132 of FIG. 1) or smart glasses (e.g., the smart glasses 133 of FIG. 1), visual, auditory, or tactile feedback information may be provided to the user.

According to an example embodiment, the user may identify a current state of a gait through the feedback information, and change how an exercise is performed based on the identified current state of the gait. For example, when the feedback information indicates that the gait speed is low, the user may increase the gait speed.

FIG. 14 is a diagram illustrating a configuration of a server according to an example embodiment.

A server 1400 may include a communicator 1410, a processor 1420, and a memory 1430. For example, the server 1400 may be the server 140 described above with reference to FIG. 1.

The communicator 1410, comprising communication circuitry, may be connected, directly or indirectly, to the processor 1420 and the memory 1430 to transmit and receive data to and from the processor 1420 and the memory 1430. The communicator 1410 may be connected to another external device to transmit and receive data to and from the external device.

The communicator 1410 may be implemented as a circuitry in the server 1400. For example, the communicator 1410 may include an internal bus and an external bus. In another example, the communicator 1410 may be an element that connects the server 1400 to the external device. The communicator 1410 may be an interface. The communicator 1410 may receive data from the external device and transmit the data to the processor 1420 and the memory 1430.

The processor 1420 may process data received from the communicator 1410 and data stored in the memory 1430. The "processor" may be a data processing device implemented by hardware including a circuit having a physical structure to perform desired operations. For example, the desired operations may include code or instructions included in a program. For example, the hardware-implemented data processing device may include a microprocessor, a central processing unit (CPU), a processor core, a multi-core processor, a multiprocessor, an application-specific integrated circuit (ASIC), and a field-programmable gate array (FPGA).

The processor 1420 may execute a computer-readable code (for example, software) stored in a memory (for example, the memory 1430) and instructions triggered by the processor 1420.

The memory 1430 may store the data received by the communicator 1410 and data processed by the processor 1420. For example, the memory 1430 may store the program (or an application, or software). The stored program may be a set of syntaxes that are coded and executable by the processor 1420 to determine one or more recommended exercise program based on an optimal value of a control parameter.

The memory 1430 may include, for example, at least one volatile memory, nonvolatile memory, random-access memory (RAM), flash memory, a hard disk drive, or an optical disc drive or combination thereof.

The memory 1430 may store an instruction set (for example, software) for operating the server 1400. The instruction set for operating the server 1400 may be executed by the processor 1420. According to an example embodiment, the memory 1430 may include a database including information on a plurality of exercise programs. According to an example embodiment, the memory 1430 may include a database storing a history of exercise programs performed by a plurality of users.

According to an example embodiment, the server 1400 may include a communication module (e.g., the communicator 1410) configured to exchange data with an external device and at least one processor (e.g., the processor(s) 1420) configured to control the server 1400, wherein the processor may be configured to receive an optimal value of a control parameter from an electronic device (e.g., the electronic device 110 of FIG. 1 or the electronic device 201 of FIG. 2), the control parameter being a parameter for adjusting at least one of a magnitude, direction and timing of a torque, an offset angle between joint angles, or sensitivity of a state factor to the joint angles or combination thereof, determine one or more recommended exercise program among a plurality of exercise programs stored in a memory (e.g., the memory 1430) of the server based on the optimal value of the control parameter, and transmit one or more recommended exercise program to the electronic device. "Based on" as used herein covers based at least on.

The processor of the server 1400 may determine the one or more recommended exercise program corresponding to the optimal value among the plurality of exercise programs stored in a memory of the server 1400 based on a history of exercise programs performed by the user.

The processor of the server 1400 may receive, from the electronic device, sensing information received by the electronic device from a wearable device while a target exercise program among the one or more recommended exercise program is being executed by the wearable device connected to the electronic device, and store the target exercise program and the sensing information in association with an account of the user.

The embodiments described herein may be implemented using a hardware component, a software component and/or a combination thereof. A processing device may be implemented using one or more general-purpose or special-purpose computers, such as, for example, a processor, a controller and an arithmetic logic unit (ALU), a DSP, a microcomputer, a field-programmable gate array (FPGA), a programmable logic unit (PLU), a microprocessor or any other device capable of responding to and executing instructions in a defined manner. The processing device may run an OS and one or more software applications that run on the OS. The processing device also may access, store, manipulate, process, and create data in response to execution of the software. For simplicity, the description of a processing device is singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors. Each "unit" herein may comprise circuitry.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs and/or DVDs; magnetooptical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher-level code that may be executed by the computer using an interpreter.

The above-described devices may be configured to act as one or more software modules in order to perform the operations of the above-described examples, or vice versa.

## Claims

1. An electronic device, comprising:
a communication module, comprising communication circuitry, configured to exchange data with an external device; and
at least one processor configured to control the electronic device,
wherein the at least one processor is configured to:
receive (810) target exercise information from a user of the electronic device, the target exercise information including at least one of target exercise time information and target exercise section information including an exercise route;
determine (820, 830) an optimal value of a control parameter that satisfies preset target values of gait assessment items based on at least one of the target exercise time information and the target exercise section information and current values of gait assessment items, wherein the optimal value is based on topographic information on the exercise route and wherein the control parameter comprising a parameter for adjusting at least one of a magnitude, direction and timing of torque, an offset angle between joint angles, or sensitivity of a state factor to the joint angles or combination thereof;
acquire (840) at least one recommended exercise program, based on the optimal value of the control parameter;
determine (850) a target exercise program among the at least one recommended exercise program;
transmit (860) information regarding the target exercise program to a wearable device worn by the user, the target exercise program configured to be executed by the wearable device;
receive (870) sensing information from the wearable device based on execution of the target exercise program; and
provide (880) feedback information to the user based on the sensing information.

2. The electronic device of claim 1, wherein
the gait assessment items comprise one or more of a step length, gait speed, gait symmetry, and gait rhythm.

3. The electronic device of claim 1, wherein
the processor is configured to acquire the current values of the gait assessment items based on previous gait information sensed while the user performs a previous exercise program.

4. The electronic device of claim 1, wherein the processor is configured to:
transmit information regarding a test exercise program for acquiring the current values of the gait assessment items to the wearable device;
receive test sensing information upon execution of the test exercise program from the wearable device; and
acquire the current values of the gait assessment items based on the test sensing information.

5. The electronic device of claim 1, wherein the processor is configured to
determine the optimal value of the control parameter such that the target values of the gait assessment items may be satisfied within a range that satisfies a set objective function.

6. The electronic device of claim 1, wherein the processor is configured to:
transmit the optimal value of the control parameter to a server; and
receive, from the server, one or more recommended exercise programs determined by the server based on the optimal value of the control parameter.

7. The electronic device of claim 1, wherein the processor is configured to
determine one or more recommended exercise programs corresponding to the optimal value among a plurality of stored exercise programs.

8. The electronic device of claim 7, wherein the processor is configured to
determine the one or more recommended exercise programs corresponding to the optimal value among the plurality of stored exercise programs based on a history of exercise programs performed by the user.

9. The electronic device of claim 1, wherein
the at least one recommended exercise program is different from a previous exercise program performed by the user on the exercise section.

10. The electronic device of claim 1, wherein the processor is configured to:
determine whether the target values of the assessment items are being satisfied based on the sensing information;
in response to the target values being not satisfied, determine a requested action to satisfy the target values; and
provide the feedback information including the requested action to the user.

11. The electronic device of claim 1, wherein the processor is configured to transmit the feedback information to an additional electronic device, and
wherein the feedback information is configured to be output by the additional electronic device.

12. The electronic device of claim 11, wherein the additional electronic device is any one of: an earphone, a glasses-type electronic device, or a watch-type electronic device.

13. The electronic device of claim 11, wherein the processor is configured to
receive a heart rate of the user from the additional electronic device as at least part of the sensing information.

14. A method performed by an electronic device, the method comprising:
receiving (810) target exercise information from a user of the electronic device, the target exercise information including at least one of target exercise time information and target exercise section information including an exercise route;
determining (820, 830) an optimal value of a control parameter that satisfies preset target values of gait assessment items based on at least one of the target exercise time information and the target exercise section information and current values of the gait assessment items, wherein the optimal value is based on topographic information on the exercise route and wherein the control parameter adjusting at least one of a magnitude, direction and timing of torque, an offset angle between joint angles, or sensitivity of a state factor to the joint angles or combination thereof;
acquiring (840) a plurality of recommended exercise programs based on the optimal value of the control parameter;
determining (850) a target exercise program among the plurality of exercise programs;
transmitting (860) information on the target exercise program to a wearable device of the user;
receiving (870) sensing information from the wearable device regarding execution of the target exercise program at the wearable device; and
providing (880) feedback information to the user based on the sensing information.

15. The method of claim 14 wherein
the at least one recommended exercise program is different from a previous exercise program performed by the user on the exercise section

## Patentansprüche

1. Elektronisches Gerät, umfassend:
ein Kommunikationsmodul, das eine Kommunikationsschaltung umfasst und dazu konfiguriert ist, Daten mit einem externen Gerät auszutauschen; und
mindestens einen Prozessor, der zum Steuern des elektronischen Geräts konfiguriert ist,
wobei der mindestens eine Prozessor dazu konfiguriert ist:
Zielübungsinformationen von einem Benutzer des elektronischen Geräts zu empfangen (810), wobei die Zielübungsinformationen mindestens eines von Zielübungszeitinformationen und Zielübungsabschnittsinformationen einschließlich einer Übungsroute umfassen;
einen optimalen Wert eines Steuerparameters zu bestimmen (820, 830), der voreingestellte Zielwerte von Gangbewertungspunkten basierend auf mindestens einem von den Zielübungszeitinformationen und den Zielübungsabschnittsinformationen sowie aktuelle Werte von Gangbewertungspunkten erfüllt, wobei der optimale Wert auf topografischen Informationen zur Übungsroute basiert, und wobei der Steuerparameter einen Parameter zum Einstellen mindestens eines von Folgendem umfasst: Magnitude, Richtung und Timing des Drehmoments, einen Versatzwinkel zwischen Gelenkwinkeln oder Empfindlichkeit eines Zustandsfaktors gegenüber den Gelenkwinkeln oder eine Kombination davon;
mindestens ein empfohlenes Übungsprogramms auf der Grundlage des optimalen Werts des Steuerparameters zu ermitteln (840);
ein Zielübungsprogramm unter dem mindestens einen empfohlenen Übungsprogramm zu bestimmen (850);
Informationen bezüglich des Zielübungsprogramms an ein vom Benutzer getragenes tragbares Gerät zu übertragen (860), wobei das Zielübungsprogramm so konfiguriert ist, dass es von dem tragbaren Gerät ausgeführt wird;
Erfassungsinformationen von dem tragbaren Gerät auf der Grundlage der Ausführung des Zielübungsprogramms zu empfangen (870); und
auf der Grundlage der Erfassungsinformationen Rückmeldeinformationen für den Benutzer bereitzustellen (880).

2. Elektronisches Gerät nach Anspruch 1, wobei
die Gangbewertungspunkte eines oder mehrere von Folgendem umfassen: eine Schrittlänge, Ganggeschwindigkeit, Gangsymmetrie und Gangrhythmus.

3. Elektronisches Gerät nach Anspruch 1, wobei
der Prozessor dazu konfiguriert ist, die aktuellen Werte der Gangbewertungspunkte auf der Grundlage früherer Ganginformationen, die während der Durchführung eines vorherigen Übungsprogramms durch den Benutzer erfasst wurden, zu ermitteln.

4. Elektronisches Gerät nach Anspruch 1, wobei der Prozessor dazu konfiguriert ist:
Informationen bezüglich eines Testübungsprogramms zum Ermitteln der aktuellen Werte der Gangbewertungspunkte an das tragbare Gerät zu übertragen;
bei Ausführung des Testübungsprogramms Test-Erfassungsinformationen vom tragbaren Gerät zu empfangen; und
die aktuellen Werte der Gangbewertungspunkte auf der Grundlage der Test-Erfassungsinformationen zu ermitteln.

5. Elektronisches Gerät nach Anspruch 1, wobei der Prozessor dazu konfiguriert ist,
den optimalen Wert des Steuerparameters zu bestimmen, so dass die Zielwerte der Gangbewertungspunkte innerhalb eines Bereichs erfüllt werden können, der eine festgelegte Zielfunktion erfüllt.

6. Elektronisches Gerät nach Anspruch 1, wobei der Prozessor dazu konfiguriert ist:
den optimalen Wert des Steuerparameters an einen Server zu übertragen; und
vom Server ein oder mehrere empfohlene Übungsprogramme zu empfangen, die vom Server auf der Grundlage des optimalen Werts des Steuerparameters bestimmt werden.

7. Elektronisches Gerät nach Anspruch 1, wobei der Prozessor dazu konfiguriert ist,
unter einer Vielzahl gespeicherter Übungsprogramme ein oder mehrere empfohlene Übungsprogramme zu bestimmen, die dem optimalen Wert entsprechen.

8. Elektronisches Gerät nach Anspruch 7, wobei der Prozessor dazu konfiguriert ist,
auf der Grundlage einer Historie der vom Benutzer durchgeführten Übungsprogramme das eine oder die mehreren empfohlenen Übungsprogramme, die dem optimalen Wert entsprechen, unter der Vielzahl gespeicherter Übungsprogramme zu bestimmen.

9. Elektronisches Gerät nach Anspruch 1, wobei
sich das mindestens eine empfohlene Übungsprogramm von einem vorherigen, vom Benutzer im Übungsabschnitt durchgeführten Übungsprogramm unterscheidet.

10. Elektronisches Gerät nach Anspruch 1, wobei der Prozessor dazu konfiguriert ist:
auf der Grundlage der Erfassungsinformationen festzustellen, ob die Zielwerte der Bewertungspunkte erfüllt werden;
als Reaktion darauf, dass die Zielwerte nicht erfüllt werden, eine angeforderte Aktion zu bestimmen, um die Zielwerte zu erfüllen; und
die Rückmeldeinformationen einschließlich der angeforderten Aktion für den Benutzer bereitzustellen.

11. Elektronisches Gerät nach Anspruch 1, wobei der Prozessor dazu konfiguriert ist, die Rückmeldeinformationen an ein zusätzliches elektronisches Gerät zu übertragen, und
wobei die Rückmeldeinformationen so konfiguriert sind, dass sie von dem zusätzlichen elektronischen Gerät ausgegeben werden.

12. Elektronisches Gerät nach Anspruch 11, wobei das zusätzliche elektronische Gerät eines von Folgendem ist: ein Hörer, ein brillenartiges elektronisches Gerät und ein uhrenartiges elektronisches Gerät.

13. Elektronisches Gerät nach Anspruch 11, wobei der Prozessor dazu konfiguriert ist,
eine Herzfrequenz des Benutzers von dem zusätzlichen elektronischen Gerät als zumindest einen Teil der Erfassungsinformationen zu empfangen.

14. Verfahren, das von einem elektronischen Gerät durchgeführt wird, wobei das Verfahren Folgendes umfasst:
Empfangen (810) von Zielübungsinformationen von einem Benutzer des elektronischen Geräts, wobei die Zielübungsinformationen mindestens eines von Zielübungszeitinformationen und Zielübungsabschnittsinformationen einschließlich einer Übungsroute umfassen;
Bestimmen (820, 830) eines optimalen Werts eines Steuerparameters, der voreingestellte Zielwerte von Gangbewertungspunkten basierend auf mindestens einem von dem Zielübungszeitinformationen und den Zielübungsabschnittsinformationen sowie aktuelle Werte der Gangbewertungspunkte erfüllt, wobei der optimale Wert auf topografischen Informationen zur Übungsroute basiert, und wobei der Steuerparameter mindestens eines von Folgendem einstellt: Magnitude, Richtung und Timing des Drehmoments, einen Versatzwinkel zwischen Gelenkwinkeln oder Empfindlichkeit eines Zustandsfaktors gegenüber den Gelenkwinkeln oder eine Kombination davon;
Ermitteln (840) einer Vielzahl empfohlener Übungsprogramme auf der Grundlage des optimalen Werts des Steuerparameters;
Bestimmen (850) eines Zielübungsprogramms unter der Vielzahl von Übungsprogrammen;
Übertragen (860) von Informationen über das Zielübungsprogramm an ein tragbares Gerät des Benutzers;
Empfangen (870) von Erfassungsinformationen vom tragbaren Gerät bezüglich der Ausführung des Zielübungsprogramms an dem tragbaren Gerät; und
Bereitstellen (880) von Rückmeldeinformationen für den Benutzer auf der Grundlage der Erfassungsinformationen.

15. Verfahren nach Anspruch 14, wobei
sich das mindestens eine empfohlene Übungsprogramm von einem vorherigen, vom Benutzer im Übungsabschnitt durchgeführten Übungsprogramm unterscheidet.

## Revendications

1. Dispositif électronique, comprenant :
un module de communication, comprenant des circuits de communication, configuré pour échanger des données avec un dispositif externe ; et
au moins un processeur configuré pour commander le dispositif électronique,
dans lequel l'au moins un processeur est configuré pour :
recevoir (810) des informations d'exercice cible d'un utilisateur du dispositif électronique, les informations d'exercice cible comprenant au moins l'une parmi des informations de temps d'exercice cible et des informations de section d'exercice cible comprenant un itinéraire d'exercice ;
déterminer (820, 830) une valeur optimale d'un paramètre de commande qui satisfait des valeurs cibles prédéfinies d'éléments d'évaluation de la démarche sur la base d'au moins l'une parmi les informations de temps d'exercice cible et les informations de section d'exercice cible et de valeurs actuelles d'éléments d'évaluation de la démarche, dans lequel la valeur optimale est basée sur des informations topographiques sur l'itinéraire d'exercice et dans lequel le paramètre de commande comprend un paramètre pour ajuster au moins l'un parmi une amplitude, une direction et un timing d'un couple, un angle de décalage entre des angles articulaires, ou une sensibilité d'un facteur d'état aux angles articulaires ou une combinaison de ceux-ci ;
acquérir (840) au moins un programme d'exercice recommandé, sur la base de la valeur optimale du paramètre de commande ;
déterminer (850) un programme d'exercice cible parmi l'au moins un programme d'exercice recommandé ;
transmettre (860) des informations concernant le programme d'exercice cible à un dispositif portable porté par l'utilisateur, le programme d'exercice cible étant configuré pour être exécuté par le dispositif portable ;
recevoir (870) des informations de détection du dispositif portable sur la base de l'exécution du programme d'exercice cible ; et
fournir (880) des informations de rétroaction à l'utilisateur sur la base des informations de détection.

2. Dispositif électronique selon la revendication 1, dans lequel
les éléments d'évaluation de la démarche comprennent un ou plusieurs parmi une longueur de pas, une vitesse de démarche, une symétrie de la démarche et un rythme de la démarche.

3. Dispositif électronique selon la revendication 1, dans lequel
le processeur est configuré pour acquérir les valeurs actuelles des éléments d'évaluation de la démarche sur la base d'informations de démarche antérieures détectées pendant que l'utilisateur exécute un programme d'exercice antérieur.

4. Dispositif électronique selon la revendication 1, dans lequel le processeur est configuré pour :
transmettre des informations concernant un programme d'exercice de test pour acquérir les valeurs actuelles des éléments d'évaluation de la démarche au dispositif portable ;
recevoir des informations de détection de test lors de l'exécution du programme d'exercice de test du dispositif portable ; et
acquérir les valeurs actuelles des éléments d'évaluation de la démarche sur la base des informations de détection de test.

5. Dispositif électronique selon la revendication 1, dans lequel le processeur est configuré pour
déterminer la valeur optimale du paramètre de commande de sorte que les valeurs cibles des éléments d'évaluation de la démarche puissent être satisfaites dans une plage qui satisfait une fonction objectif définie.

6. Dispositif électronique selon la revendication 1, dans lequel le processeur est configuré pour :
transmettre la valeur optimale du paramètre de commande à un serveur ; et
recevoir, du serveur, un ou plusieurs programmes d'exercice recommandés déterminés par le serveur sur la base de la valeur optimale du paramètre de commande.

7. Dispositif électronique selon la revendication 1, dans lequel le processeur est configuré pour
déterminer un ou plusieurs programmes d'exercice recommandés correspondant à la valeur optimale parmi une pluralité de programmes d'exercice stockés.

8. Dispositif électronique selon la revendication 7, dans lequel le processeur est configuré pour
déterminer le ou les programmes d'exercice recommandés correspondant à la valeur optimale parmi la pluralité de programmes d'exercice stockés sur la base d'un historique de programmes d'exercice exécutés par l'utilisateur.

9. Dispositif électronique selon la revendication 1, dans lequel
l'au moins un programme d'exercice recommandé est différent d'un programme d'exercice antérieur exécuté par l'utilisateur sur la section d'exercice.

10. Dispositif électronique selon la revendication 1, dans lequel le processeur est configuré pour :
déterminer si les valeurs cibles des éléments d'évaluation sont satisfaites sur la base des informations de détection ;
en réponse à la non-satisfaction des valeurs cibles, déterminer une action requise pour satisfaire les valeurs cibles ; et
fournir les informations de rétroaction comprenant l'action requise à l'utilisateur.

11. Dispositif électronique selon la revendication 1, dans lequel le processeur est configuré pour transmettre les informations de rétroaction à un dispositif électronique supplémentaire, et
dans lequel les informations de rétroaction sont configurées pour être émises par le dispositif électronique supplémentaire.

12. Dispositif électronique selon la revendication 11, dans lequel le dispositif électronique supplémentaire est l'un quelconque parmi : un écouteur, un dispositif électronique de type lunettes ou un dispositif électronique de type montre.

13. Dispositif électronique selon la revendication 11, dans lequel le processeur est configuré pour
recevoir une fréquence cardiaque de l'utilisateur du dispositif électronique supplémentaire en tant qu'au moins une partie des informations de détection.

14. Procédé exécuté par un dispositif électronique, le procédé comprenant :
recevoir (810) des informations d'exercice cible d'un utilisateur du dispositif électronique, les informations d'exercice cible comprenant au moins l'une parmi des informations de temps d'exercice cible et des informations de section d'exercice cible comprenant un itinéraire d'exercice ;
déterminer (820, 830) une valeur optimale d'un paramètre de commande qui satisfait des valeurs cibles prédéfinies d'éléments d'évaluation de la démarche sur la base d'au moins l'une parmi les informations de temps d'exercice cible et les informations de section d'exercice cible et de valeurs actuelles des éléments d'évaluation de la démarche, dans lequel la valeur optimale est basée sur des informations topographiques sur l'itinéraire d'exercice et dans lequel le paramètre de commande ajuste au moins l'un parmi une amplitude, une direction et un timing d'un couple, un angle de décalage entre des angles articulaires, ou une sensibilité d'un facteur d'état aux angles articulaires ou une combinaison de ceux-ci ;
acquérir (840) une pluralité de programmes d'exercice recommandés sur la base de la valeur optimale du paramètre de commande ;
déterminer (850) un programme d'exercice cible parmi la pluralité de programmes d'exercice ;
transmettre (860) des informations sur le programme d'exercice cible à un dispositif portable de l'utilisateur ;
recevoir (870) des informations de détection du dispositif portable concernant l'exécution du programme d'exercice cible sur le dispositif portable ; et
fournir (880) des informations de rétroaction à l'utilisateur sur la base des informations de détection.

15. Procédé selon la revendication 14, dans lequel
l'au moins un programme d'exercice recommandé est différent d'un programme d'exercice antérieur exécuté par l'utilisateur sur la section d'exercice.
